(19) Europäisches Patentamt
European Patent Office
Office européen des brevets

(11) EP 4 521 410 A1

(12) EUROPEAN PATENT APPLICATION

(43) Date of publication:
12.03.2025 Bulletin 2025/11

(51) International Patent Classification (IPC):
G16B 15/20 (2019.01)

(21) Application number: 24193053.6

(22) Date of filing: 06.08.2024

(52) Cooperative Patent Classification (CPC):
G16B 15/20

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 11.09.2023 JP 2023146663

(71) Applicant: FUJITSU LIMITED
Kawasaki-shi, Kanagawa 211-8588 (JP)

(72) Inventor: Katayama, Kentaro
Kawasaki-shi, Kanagawa, 211-8588 (JP)

(74) Representative: Hoffmann Eitle
Patent- und Rechtsanwälte PartmbB
Arabellastraße 30
81925 München (DE)

(54) **STABLE CONFORMATION SEARCH SYSTEM, STABLE CONFORMATION SEARCH METHOD, AND STABLE CONFORMATION SEARCH PROGRAM**

(57) A system performs: obtaining a model where N (N: an integer of > 1) main chain particles in a sequence and N side chain particles correspondingly linked to the respective N main chain particles are arranged in a lattice space; calculating coordinates of an (i+1)th main chain particle, by using coordinates of an i-th main chain particle and a first state variable represented by relative coordinates between the i-th and (i+1)th main chain particles; calculating coordinates of an (i+1)th side chain particle corresponding to the (i+1)th main chain particle, by using the coordinates of the (i+1)th main chain particle and a second state variable represented by relative coordinates between the (i+1)th main and side chain particles; calculating a value of an energy of the model when any one of the first and second state variables is changed; and identifying the first and second state variables having a local minimum value.

FIG. 1

**Description**

FIELD

The embodiments discussed herein are related to a stable conformation search system, a stable conformation search method, and a stable conformation search program.

BACKGROUND

In recent years, in the field of drug discovery, middle molecules (molecular weights of 500 to 3000) with few side effects have been attracting attention, and development of a search method for searching for a stable conformation of middle molecules has been advancing.

As an example, the present applicant has studied a search method for searching for a stable conformation of a coarse-grained model by using interaction potentials between coarse-grained particles. For example, the present applicant has studied the search method for reducing the amount of calculation to search for a stable conformation of the coarse-grained model.

Japanese Laid-open Patent Publication No. 2021-82165 is disclosed as related art.

SUMMARY

TECHNICAL PROBLEM

However, the above-described search method in the example is intended to search for a stable conformation of main chain particles, and performs the search without considering characteristics of side chain particles.

According to one aspect, an object is to further improve search accuracy in searching for a stable conformation of a model.

SOLUTION TO PROBLEM

According to an aspect of the embodiments, there is provided a stable conformation search system including: a first calculation unit configured to obtain a model in which N (N is an integer of two or more) main chain particles arranged in a sequence and N side chain particles correspondingly linked to the respective N main chain particles are arranged in a lattice space, and calculate coordinates of an (i+1)th main chain particle in the sequence in the lattice space, by using coordinates of an i-th main chain particle in the sequence in the lattice space and a first state variable represented by relative coordinates between the i-th main chain particle and the (i+1)th main chain particle in the lattice space; a second calculation unit configured to calculate coordinates of an (i+1)th side chain particle corresponding to the (i+1)th main chain particle in the lattice space, by using the coordinates of the (i+1)th main chain particle in the lattice space and a second state variable represented by relative coordinates between the (i+1)th main chain particle and the (i+1)th side chain particle; a calculation unit configured to calculate a value of an energy of the model in a case where any one of the first state variable and the second state variable is changed; and an identification unit configured to identify the first state variable and the second state variable with which the value of the energy is a local minimum value.

ADVANTAGEOUS EFFECTS OF INVENTION

Search accuracy in searching for a stable conformation of a model may be further improved.

BRIEF DESCRIPTION OF DRAWINGS

FIG. 1 is a diagram illustrating an application example of a stable conformation search system;
FIG. 2 is a diagram illustrating an outline of stable conformation search processing for a two bead model;
FIG. 3 (i.e., FIGs. 3A and 3B) is a diagram illustrating a representation method using relative coordinates;
FIG. 4A is a diagram illustrating a specific example of a first state variable;
FIG. 4B is a diagram illustrating a specific example of a second state variable;
FIG. 5 is a diagram illustrating details of energy calculation processing;
FIG. 6 is a first diagram illustrating details of terms in the energy calculation processing;
FIG. 7 is a second diagram illustrating the details of the terms in the energy calculation processing;

FIG. 8 is a third diagram illustrating the details of the terms in the energy calculation processing;

FIG. 9 is a diagram illustrating the magnitude of attractive force between amino acid residues;

FIG. 10 is a diagram illustrating an example of a system configuration of the stable conformation search system and a functional configuration of a terminal apparatus;

FIG. 11 is a diagram illustrating an example of the system configuration of the stable conformation search system and a functional configuration of an Ising apparatus;

FIG. 12 is a diagram illustrating a specific example of processing by the Ising apparatus;

FIG. 13 is a diagram illustrating an example of a hardware configuration of the terminal apparatus;

FIG. 14 is a flowchart illustrating a sequence of stable conformation search processing;

FIG. 15 is a flowchart illustrating details of the search processing;

FIG. 16 is a flowchart illustrating details of determination processing; and

FIG. 17 is a diagram illustrating an example of an effect of reducing the amount of calculation.

DESCRIPTION OF EMBODIMENTS

The embodiments will be described below with reference to the accompanying drawings. In the present specification and drawings, constituent elements having substantially the same functional configurations will be denoted by the same signs, and redundant description thereof will be omitted.

[First Embodiment]

<APPLICATION EXAMPLE OF STABLE CONFORMATION SEARCH SYSTEM>

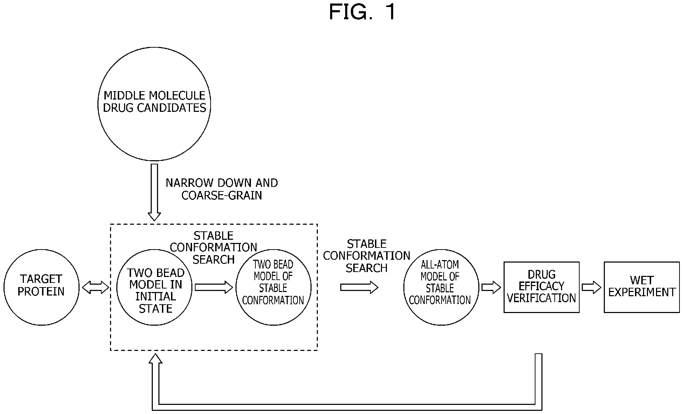

First, description is given of an application example of a stable conformation search system according to a first embodiment. FIG. 1 is a diagram illustrating the application example of the stable conformation search system.

In the field of drug discovery, generally, use of small molecules has been a mainstream so far. However, a small molecule is likely to bind to a protein other than a target protein, and has a possibility of inhibiting a function other than a target function. For this reason, middle molecules with few side effects have been attracting attention in recent years.

In the case of a middle molecule as a drug candidate, a search for a stable conformation involves an enormous amount of calculation. Accordingly, the present applicant has been developing a search method including first narrowing down to a specific middle molecule (for example, a cyclic peptide) and then searching for a stable conformation of the narrowed middle molecule in the following two phases.

The first phase is a phase of generating a two bead model and searching for a stable conformation of the two bead model. The two bead model is a model in which one amino acid residue is represented by two types of particles (coarse-grained particles). Hereinafter, multiple atoms (atom groups) for forming amino bonds among atom groups included in amino acid residues are referred to as main chain particles. Multiple atoms (atom groups) that are other than the atoms for forming the amino bonds among the atom groups included in the amino acid residues and that are located in side chains corresponding to the main chain particles are referred to as side chain particles. The stable conformation search system according to the first embodiment is a system that is applied to the first phase indicated by a dotted frame in FIG. 1, and that searches for a stable conformation of a two bead model.

The second phase is a phase of searching for a stable conformation of all the atoms based on the two bead model of the searched-out stable conformation, and further verifying a drug efficacy based on the all-atom model of the searched-out stable conformation.

In a case where a desired drug efficacy is not obtained in the second phase, the method returns to the first phase, and further searches for a stable conformation of the two bead model. The above cycle of these phases is repeated until the desired drug efficacy is obtained. When the desired drug efficacy is obtained, for example, a pharmaceutical maker synthesizes a middle-molecular drug based on the all-atom model of the stable conformation at that time, and performs an activity evaluation or the like (so-called wet experiment) thereon.

The stable conformation search system according to the first embodiment, which will be described in detail below, is a system applied to the first phase in a series of sequences of middle molecule drug discovery as described above.

<OUTLINE OF STABLE CONFORMATION SEARCH PROCESSING OF TWO BEAD MODEL>

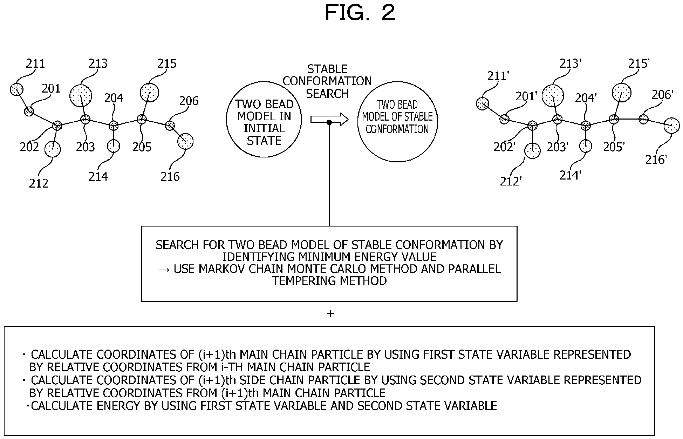

Next, an outline of stable conformation search processing of a two bead model by the stable conformation search system according to the first embodiment will be described. FIG. 2 is a diagram illustrating the outline of the stable conformation search processing of the two bead model.

The stable conformation search system according to the first embodiment generates a two bead model as amino acid molecules linked in a linear chain by arranging, in a sequence in a space (lattice space) of, for example, a face-centered cubic lattice (FCC): [a] skeleton moieties (reference signs 201 to 206 in FIG. 2) each centered on an $\alpha$ carbon of an amino acid forming a main chain particle of a peptide, and [b] side chain particles (reference signs 211 to 216 in FIG. 2). The stable conformation search system searches for a stable conformation of the generated two bead model as a combinatorial optimization problem by using an Ising apparatus.

As illustrated in FIG. 2, when searching for a stable conformation of a two bead model, the stable conformation search system according to the first embodiment iterates a state transition of the two bead model by: [a] moving the position of each main chain particle in the space of the face-centered cubic lattice, and [b] moving the position of the corresponding side chain particle to a position corresponding to the position to which the main chain particle is moved in the space of the face-centered cubic lattice. In the process of iterating the state transition, the stable conformation search system according to the first embodiment determines whether each transition (also referred to change) is acceptable or not based on a difference between an energy value before the transition and an energy value after the transition, and identifies the minimum energy value (to be precise, the local minimum value of the energy value) among the energy values after the transitions (after the changes) each of which is determined as acceptable. In this way, the stable conformation search system according to the first embodiment searches for the two bead model of the stable conformation.

For example, the stable conformation search system according to the first embodiment identifies the minimum energy value without falling into a local solution by using the Markov-Chain Monte Carlo (MCMC) method to determine that the transition is acceptable if the transition satisfies a predetermined condition, for example, [a] determining that a transition is acceptable if the energy value after the transition is smaller than the energy value before the transition, and [b] determining that a transition is acceptable if a difference between the energy value after the transition and the energy value before the transition is smaller than a predetermined thermal noise even when the energy value after the transition is larger than the energy value before the transition.

The stable conformation search system according to the first embodiment efficiently identifies the minimum energy value by iterating the state transition of the two bead model using the parallel tempering method (PT).

The stable conformation search system according to the first embodiment identifies the minimum energy value with a small amount of calculation by:

[a] calculating the coordinates of an (i+1)th main chain particle in a sequence among multiple main chain particles arranged in the sequence in the space of the face-centered cubic lattice by using a first state variable represented by relative coordinates from the coordinates of the i-th main chain particle in the sequence,

[b] calculating the coordinates of an (i+1)th side chain particle by using a second state variable represented by relative coordinates from the coordinates of the (i+1)th main chain particle in the sequence, and

[c] calculating the energy value of the two bead model every time any one of the first state variable and the second state variable is changed (details of an energy value calculation method will be described later).

The "first state variable" and the "second state variable" herein are variables for specifying a state of the two bead model (the coordinates of each main chain particle and the coordinates of each side chain particle of the two bead model).

In FIG. 2, reference signs 201' to 206' and 211' to 216' schematically illustrate a stable conformation of the two bead model (the main chain particles and the side chain particles) that the stable conformation search system according to the first embodiment searches out by identifying the minimum energy value.

<ADVANTAGE OF REPRESENTING FIRST AND SECOND STATE VARIABLES BY RELATIVE COORDINATES>

As described above, the stable conformation search system according to the first embodiment calculates the coordinates of the (i+1)th main chain particle by using the first state variable represented by the relative coordinates from the coordinates of the i-th main chain particle. The stable conformation search system according to the first embodiment calculates the coordinates of the (i+1)th side chain particle by using the second state variable represented by the relative coordinates from the coordinates of the (i+1)th main chain particle. The advantage of representing the state variables by the relative coordinates will be described below.

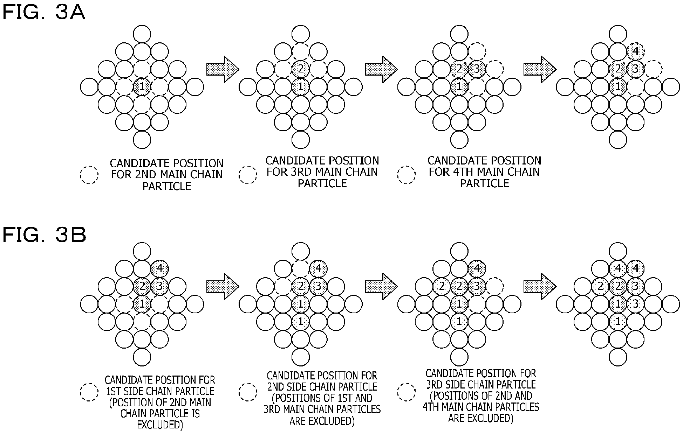

FIGs. 3A and 3B are diagrams illustrating a representation method using relative coordinates. In an example illustrated in FIGs. 3A and 3B, the space of the face-centered cubic lattice is represented by a two-dimensional plane for simplification of description.

FIG. 3A illustrates a case where the first state variable is represented by relative coordinates. As illustrated in FIG. 3A, when the position of the first main chain particle included in the two bead model is determined, the number of candidate positions for the second main chain particle adjacent to the first main chain particle is four.

When the position of the second main chain particle included in the two bead model is determined, the number of candidate positions for the third main chain particle adjacent to the candidate position of the second main chain particle is three. When the position of the third main chain particle included in the two bead model is determined, the number of candidate positions for the fourth main chain particle adjacent to the candidate position of the third main chain particle is three.

As described above, in the case where the coordinates of each main chain particle are specified by using the first state variable represented by the relative coordinates, the number of candidate positions for an (i+1)th main chain particle is equal to or smaller than a certain number regardless of the number of main chain particles included in the two bead model, and thus the number of bits of the first state variable does not increase.

For example, when the first state variable is represented by the relative coordinates as illustrated in FIG. 3A, there is an advantage that the number of bits of the first state variable may be reduced. In finding a solution to the problem replaced with the Ising model, the number of parameters such as the first state variables increases as the problem scale becomes larger. For this reason, the efficiency of the operation may decrease because there is a possibility that all parameters used in the solution finding are not allowed to be stored in a storage unit used as a cache by an arithmetic unit that executes solution finding. As described above, the advantage to reduce the number of bits of the first state variable makes it easier to secure the storage capacity needed to store the various parameters required for the solution, thereby improving the efficiency of the calculation.

Similarly, FIG. 3B illustrates a case where a second state variable is represented by relative coordinates. As illustrated in FIG. 3B, when the position of the first main chain particle included in the two bead model is determined, the number of candidate positions for a first side chain particle adjacent to the first main chain particle is three except for the position of the second main chain particle.

When the position of the second main chain particle included in the two bead model is determined, the number of candidate positions for the second side chain particle adjacent to the candidate position of the second main chain particle is two except for the positions of the first and third main chain particles. When the position of the third main chain particle included in the two bead model is determined, the number of candidate positions for the third side chain particle adjacent to the candidate position of the third main chain particle is two except for the positions of the second and fourth main chain particles.

As described above, in the case where the coordinates of each side chain particle are specified by using the second state variable represented by the relative coordinates, the number of candidate positions for an i-th side chain particle when the coordinates of an i-th main chain particle are determined is equal to or smaller than a certain number regardless of the number of side chain particles included in the two bead model. For this reason, the number of bits of the second state variable does not increase either.

For example, when the second state variable is represented by the relative coordinates as illustrated in FIG. 3B, there is an advantage that the number of bits of the second state variable may be reduced. In finding a solution to the problem replaced with the Ising model, the number of parameters such as the second state variables increases as the problem scale becomes larger. For this reason, the efficiency of the operation may decrease because there is a possibility that all parameters used in the solution finding are not allowed to be stored in a storage unit used as a cache by an arithmetic unit that executes solution finding. As described above, the advantage to reduce the number of bits of the second state variable makes it easier to secure the storage capacity needed to store the various parameters required for the solution, thereby improving the efficiency of the calculation.

<SPECIFIC EXAMPLES OF FIRST AND SECOND STATE VARIABLES>

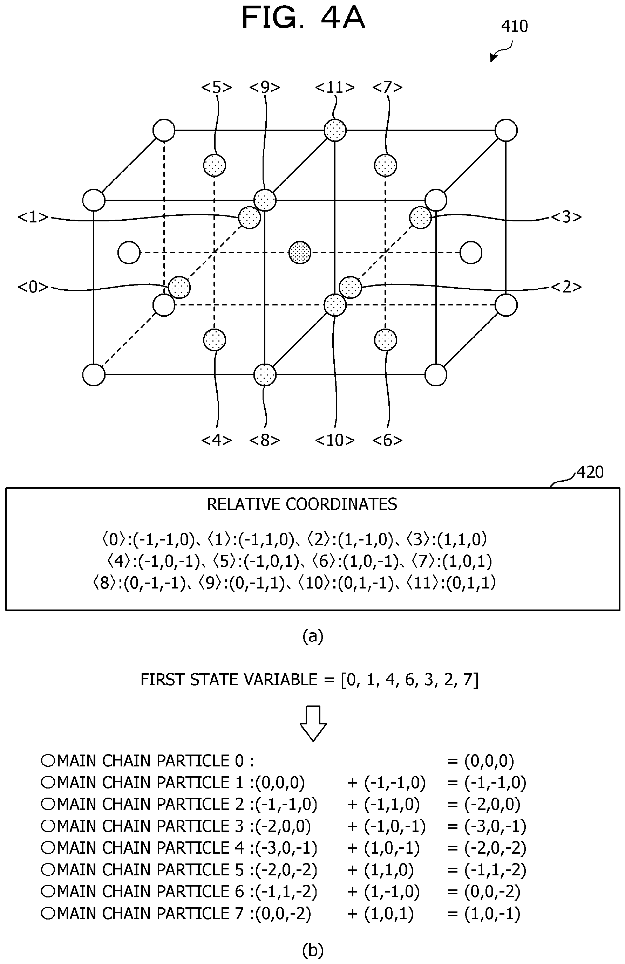

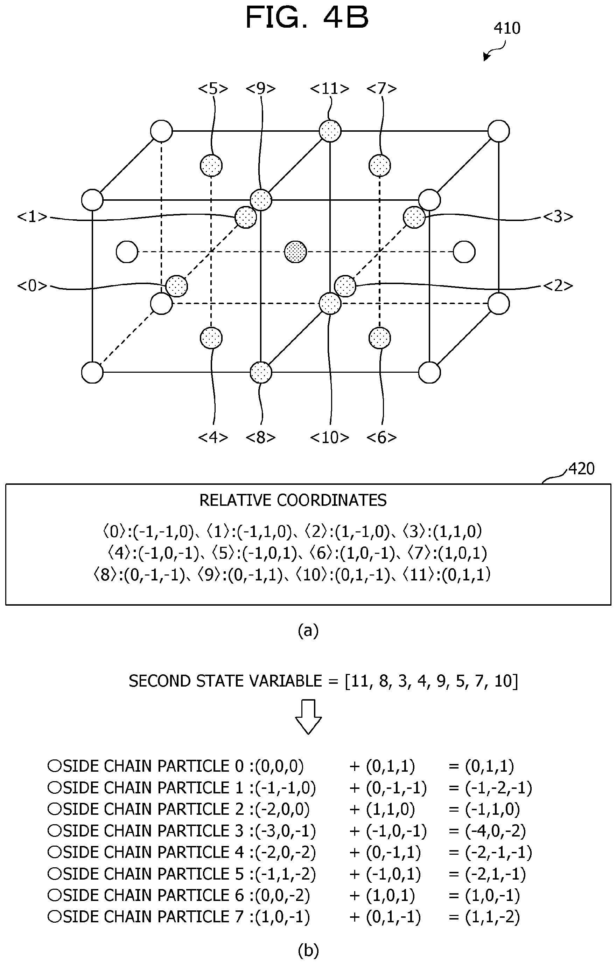

Next, specific examples of the first state variable and the second state variable represented by the relative coordinates will be described. FIGs. 4A and 4B are diagrams illustrating the specific examples of the first and second state variables.

As illustrated in FIG. 4A, the center position of a face-centered cubic lattice (FCC) 410 is the origin (coordinates = (0, 0, 0)), and an i-th main chain particle is located at the origin. In this case, the number of candidate positions for the (i+1)th main chain particle adjacent to the i-th main chain particle is 12 (see <0> to <11>). The relative coordinates of the 12 candidate positions for the main chain particle from the origin are as follows.

Relative Coordinates of Position <0>: (-1, -1, 0)
Relative Coordinates of Position <1>: (-1, 1, 0)
Relative Coordinates of Position <2>: (1, -1, 0)
Relative Coordinates of Position <3>: (1, 1, 0)
Relative Coordinates of Position <4>: (-1, 0, -1)
Relative Coordinates of Position <5>: (-1, 0, 1)
Relative Coordinates of Position <6>: (1, 0, -1)
Relative Coordinates of Position <7>: (1, 0, 1)
Relative Coordinates of Position <8>: (0, -1, -1)
Relative Coordinates of Position <9>: (0, -1, 1)
Relative Coordinates of Position <10>: (0, 1, -1)

Relative Coordinates of Position <11>: (0, 1, 1)

For example, the coordinates of the (i+1)th main chain particle adjacent to the i-th main chain particle are the relative coordinates of any of the above 12 candidate positions (see reference sign 420).

As an example, (b) of FIG. 4A presents an example of a two bead model including eight main chain particles in which the coordinates of each of the main chain particles are calculated by using the first state variable represented by the relative coordinates. For example, when the coordinates of the zeroth main chain particle are set at the origin (coordinates = (0, 0, 0)) and the first state variable is set as [0, 1, 4, 6, 3, 2, 7], it is possible to calculate the coordinates as follows:

- the coordinates of the first main chain particle = the coordinates of the zeroth main chain particle + the relative coordinates of the position <0>;
- the coordinates of the second main chain particle = the coordinates of the first main chain particle + the relative coordinates of the position <1>;
- the coordinates of the third main chain particle = the coordinates of the second main chain particle + the relative coordinates of the position <4>;
- the coordinates of the fourth main chain particle = the coordinates of the third main chain particle + the relative coordinates of the position <6>;
- the coordinates of the fifth main chain particle = the coordinates of the fourth main chain particle + the relative coordinates of the position <3>;
- the coordinates of the sixth main chain particle = the coordinates of the fifth main chain particle + the relative coordinates of the position <2>; and
- the coordinates of the seventh main chain particle = the coordinates of the sixth main chain particle + the relative coordinates of the position <7>.

Note that the number of the values included in the first state variable is (the number of the main chain particles - 1) as described above.

Similarly, as illustrated in (a) of FIG. 4B, the center position of the face-centered cubic lattice (FCC) 410 is the origin (coordinates = (0, 0, 0)) and an i-th main chain particle is located at the origin. In this case, the number of candidate positions for the i-th side chain particle adjacent to the i-th main chain particle is 12 (see <0> to <11>). The relative coordinates of the 12 candidate positions for the side chain particle from the origin are as follows.

Relative Coordinates of Position <0>: (-1, -1, 0)
Relative Coordinates of Position <1>: (-1, 1, 0)
Relative Coordinates of Position <2>: (1, -1, 0)
Relative Coordinates of Position <3>: (1, 1, 0)
Relative Coordinates of Position <4>: (-1, 0, -1)
Relative Coordinates of Position <5>: (-1, 0, 1)
Relative Coordinates of Position <6>: (1, 0, -1)
Relative Coordinates of Position <7>: (1, 0, 1)
Relative Coordinates of Position <8>: (0, -1, -1)
Relative Coordinates of Position <9>: (0, -1, 1)
Relative Coordinates of Position <10>: (0, 1, -1)
Relative Coordinates of Position <11>: (0, 1, 1)

For example, the coordinates of the i-th sub chain particle adjacent to the i-th main chain particle are the relative coordinates of any of the above 12 candidate positions (see reference sign 420).

As an example, (b) of FIG. 4B presents an example of a two bead model including eight main chain particles in which the positions of all the main chain particles are determined and the coordinates of the side chain particle corresponding to each main chain particle are calculated by using the second state variable represented by the relative coordinates. For example, when the second state variable = [11, 8, 3, 4, 9, 5, 7, 10] is set relative to the coordinates of the zeroth to seventh main chain particles, it is possible to calculate the coordinates as follows:

- the coordinates of the zeroth side chain particle = the coordinates of the zeroth main chain particle + the relative coordinates of the position <11>;
- the coordinates of the first side chain particle = the coordinates of the first main chain particle + the relative coordinates of the position <8>;
- the coordinates of the second side chain particle = the coordinates of the second main chain particle + the relative coordinates of the position <3>;

- the coordinates of the third side chain particle = the coordinates of the third main chain particle + the relative coordinates of the position <4>;
- the coordinates of the fourth side chain particle = the coordinates of the fourth main chain particle + the relative coordinates of the position <9>;
- the coordinates of the fifth side chain particle = the coordinates of the fifth main chain particle + the relative coordinates of the position <5>;
- the coordinates of the sixth side chain particle = the coordinates of the sixth main chain particle + the relative coordinates of the position <7>; and
- the coordinates of the seventh side chain particle = the coordinates of the seventh main chain particle + the relative coordinates of the position <10>.

Note that the number of the values included in the second state variable is equal to (the number of main chain particles) as described above.

<STATE TRANSITIONS OF TWO BEAD MODEL>

Next, an example of state transitions of the two bead model will be described. In the first embodiment, a state transition of the two bead model is performed by moving the main chain particles as follows:

- a state $S_i$ (i = 2 to N) is selected and is transitioned to another state different from the state $S_i$ by moving the i-th main chain particle.;
- the i-th main chain particle may be moved randomly or may be moved such that the first state variable is incremented by one; and
- as the i-th main chain particle is moved, the (i+1)th to N-th main chain particles and the i-th to N-th side chain particles are translated together with the i-th main chain particle.

In the first embodiment, a state transition of the two bead model is performed by moving the side chain particles as follows:

- a state $R_i$ (i = 2 to N) is selected and is transitioned to another state different from the state $R_i$;
- the i-th side chain particle may be selected arbitrarily or may be moved when the i-th main chain particle is moved;
- the i-th side chain particle may be moved randomly or may be moved such that the second state variable is incremented by one; and
- the i-th side chain particle is moved to a destination at which the i-th side chain particle may not collide with another main or side chain particle,
- where, for example, the coordinates of the movement destination of the i-th side chain particle are selected so as not to coincide with the coordinates of all the main chain particles and the coordinates of all the side chain particles other than the i-th side chain particle, or
- where the coordinates of the movement destination of the i-th side chain particle are selected so as not to coincide with the coordinates of the main chain particle adjacent to the i-th main chain particle (which is the (i-1)th main chain particle or the (i+1)th main chain particle).

As described above, in the first embodiment, the state transitions in consideration of the side chain particles are performed.

<DETAILS OF ENERGY CALCULATION PROCESSING>

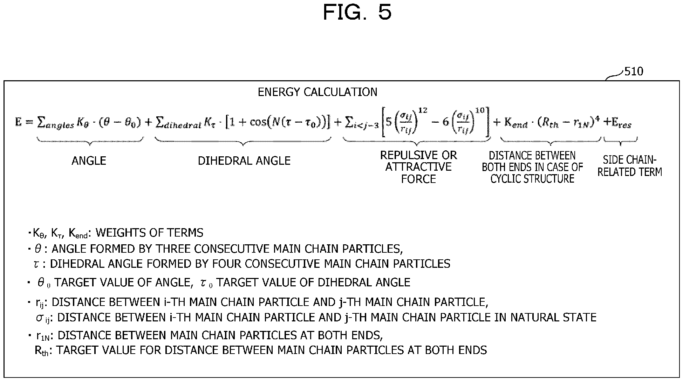

Next, description will be given of details of energy calculation processing that the stable conformation search system according to the first embodiment executes based on interactions between main chain particles, interactions between the main chain particles and side chain particles, and the like, in searching for the stable conformation of the two bead model. FIG. 5 is a diagram illustrating the details of the energy calculation processing.

As illustrated in FIG. 5, the energy calculation processing (see reference sign 510) executed in searching for the stable conformation of the two bead model includes:

- a process for calculating energies based on interactions between main chain particles, the energies including: an energy depending on an angle among main chain particles, an energy depending on a dihedral angle among main chain particles, an energy depending on a repulsive force or attractive force between main chain particles, and an energy depending on a distance between main chain particles at both ends in a case where the two bead model has a cyclic structure; and

- a process for calculating a side chain particle-related energy,

and is expressed by, for example, Formula 1 below:

For example, the energy calculation processing is expressed by Formula 1 below:

$$E = \sum_{angles} K_\theta \cdot (\theta - \theta_0) + \sum_{dihedral} K_\tau \cdot \left[1 + \cos\big(N(\tau - \tau_0)\big)\right] +$$
$$\sum_{i<j-3} \left[5 \left(\frac{\sigma_{ij}}{r_{ij}}\right)^{12} - 6 \left(\frac{\sigma_{ij}}{r_{ij}}\right)^{10}\right] + K_{end} \cdot (R_{th} - r_{1N})^4 + E_{res}$$

... (Formula 1),

where $K_\theta$, $K_\tau$, and $K_{end}$ denote weights of respective terms. In Formula 1 above: $\theta$ denotes an angle formed by three consecutive (i-1)th to (i+1)th main chain particles; $\tau$ denotes a dihedral angle formed by four consecutive (i-2)th to (i+1)th main chain particles; $\theta_0$ denotes a target value of the angle; $\tau_0$ denotes a target value of the dihedral angle; $r_{ij}$ denotes a distance between the i-th main chain particle and a j-th main chain particle; $\sigma_{ij}$ denotes a distance between the i-th main chain particle and the j-th main chain particle in a natural state; $r_{1N}$ denotes a distance between the main chain particles at both ends; $R_{th}$ denotes a target value of the distance between the main chain particles at both ends; and $E_{res}$ denotes an energy term related to the side chain particles.

In order to execute the energy calculation processing described above, the stable conformation search system according to the first embodiment calculates, as a calculation target, a difference between the energy values generated with movement of the position of each main chain particle. Thus, the stable conformation search system according to the first embodiment makes it possible to reduce the amount of calculation in the energy calculation processing.

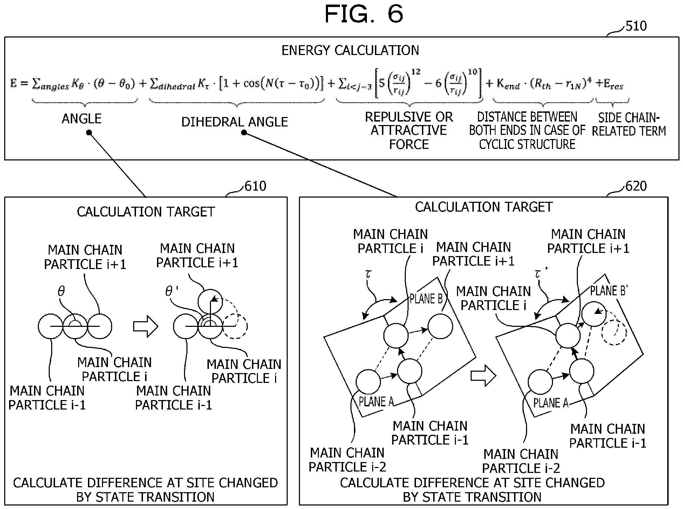

FIGs. 6 to 8 are first to third diagrams illustrating details of respective terms in the energy calculation processing. As indicated with reference sign 610 in FIG. 6, when calculating the energy depending on the angle among the main chain particles, the stable conformation search system according to the first embodiment calculates, as the calculation target, the difference between the energy values generated with the movement of the position of each main chain particle.

For example, in a case where a state transition of the two bead model is made by moving an (i+1)th main chain particle relative to the i-th main chain particle, the stable conformation search system calculates, as the energy depending on the angle among the main chain particles, an energy depending on a difference (= $\theta$ - $\theta'$) between:

- the angle ($\theta$) formed by the (i-1)th, i-th, and (i+1)th main chain particles before the movement of the (i+1)th main chain particle (the angle $\theta$ centered on the i-th main chain particle before the movement of the (i+1)th main chain particle), and
- the angle ($\theta'$) formed by the (i-1)th, i-th, and (i+1)th main chain particles after the movement of the (i+1)th main chain particle (the angle $\theta'$ centered on the i-th main chain particle after the movement of the (i+1)th main chain particle).

For example, the angle centered on the (i-1)th main chain particle, the angle centered on the (i-2)th main chain particle, ..., and so on remain unchanged before and after the movement of the (i+1)th main chain particle, and therefore may be excluded from the calculation of the energy depending on the angle among the main chain particles. All the (i+2)th and subsequent main chain particles are translated while maintaining their angles when the (i+1)th main chain particle is moved. For example, the angle centered on the (i+1)th main chain particle, the angle centered on the (i+2)th main chain particle, ..., and so on remain unchanged before and after the movement of the (i+1)th main chain particle, and therefore may be excluded from the calculation of the energy depending on the angle among the main chain particles.

Similarly, as indicated with reference sign 620 in FIG. 6, when calculating the energy depending on the dihedral angle among the main chain particles, the stable conformation search system according to the first embodiment calculates, as a calculation target, a difference between the energy values generated with movement of the position of each main chain particle.

For example, in a case where a state transition of the two bead model is made by moving an (i+1)th main chain particle relative to the i-th main chain particle, the stable conformation search system calculates, as the energy depending on the dihedral angle among the main chain particles, an energy depending on a difference (= $\tau$ - $\tau'$) between:

- a dihedral angle $\tau$ formed between a plane A formed by the (i-2)th to i-th main chain particles and a plane B formed by the (i-1)th to (i+1)th main chain particles before the movement of the (i+1)th main chain particle, and
- a dihedral angle $\tau'$ formed between the plane A formed by the (i-2)th to i-th main chain particles and a plane B' formed by the (i-1)th to (i+1)th main chain particles after the movement of the (i+1)th main chain particle.

For example, a dihedral angle formed between a plane formed by the (i-3)th to (i-1)th main chain particles and a plane formed by the (i-2)th to i-th main chain particles and so on remain unchanged before and after the movement of the (i+1)th main chain particle. For this reason, the above dihedral angle may be excluded from the calculation of the energy depending on the dihedral angle among the main chain particles. All the (i+2)th and subsequent main chain particles are translated while maintaining their angles when the (i+1)th main chain particle is moved. For example, a dihedral angle formed between the plane formed by the (i-1)th to (i+1)th main chain particles and a plane formed by the i-th to (i+2)th main chain particles and so on remain unchanged before and after the movement of the (i+1)th main chain particle. For this reason, the above dihedral angle may be excluded from the calculation of the energy depending on the dihedral angle among the main chain particles.

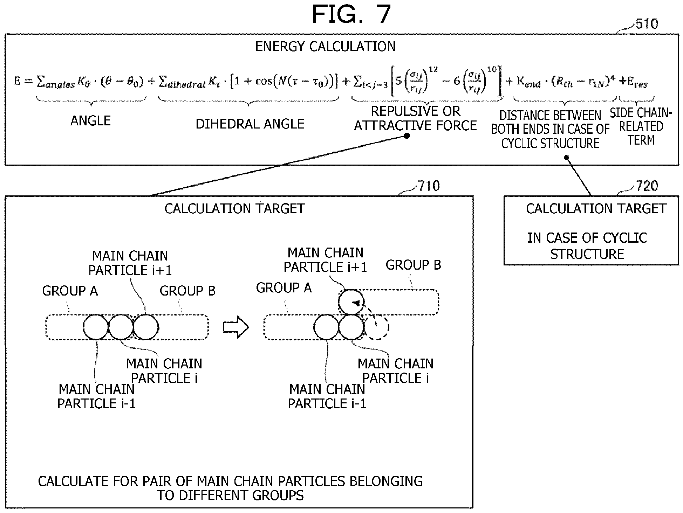

As indicated with reference sign 710 in FIG. 7, when calculating the energy depending on the repulsive force or attractive force between the main chain particles, the stable conformation search system according to the first embodiment sets, as calculation targets, all pairs of main chain particles belonging to different groups. For example, the repulsive force or attractive force between the main chain particles belonging to the same group may be excluded from the energy calculation.

For example, in a case where a state transition of the two bead model is made by moving an (i+1)th main chain particle relative to the i-th main chain particle, the stable conformation search system calculates, as the energy depending on the repulsive force or attractive force between the main chain particles, an energy depending on a difference between:

- the repulsive force or attractive force calculated for all the pairs of the main chain particles belonging to a group A (group including the first to i-th main chain particles) and the main chain particles belonging to a group B (group including the (i+1)th and subsequent main chain particles) before the movement of the (i+1)th main chain particle, and
- the repulsive force or attractive force calculated for all the pairs of the main chain particles belonging to the group A (group including the first to i-th main chain particles) and the main chain particles belonging to the group B (group including the (i+1)th and subsequent main chain particles) after the movement of the (i+1)th main chain particle.

For example, the energy depending on the repulsive force or attractive force between the main chain particles belonging to the group A remains unchanged before and after the movement of the (i+1)th main chain particle, and therefore may be excluded from the calculation of the energy depending on the repulsive force or attractive force between the main chain particles. Since the main chain particles belonging to the group B are translated while maintaining their distances in between when the (i+1)th main chain particle is moved, the repulsive force or attractive force between the main chain particles belonging to the group B remains unchanged before and after the movement of the (i+1)th main chain particle. For this reason, the energy depending on the above repulsive force or attractive force may be excluded from the calculation of the energy depending on the repulsive force or the attractive force between the main chain particles.

As indicated with reference sign 720 in FIG. 7, in a case where the two bead model has a cyclic structure, whenever a state transition of the two bead model is made by moving an (i+1)th main chain particle relative to the i-th main chain particle, the distance between the main chain particles at both ends changes.

For this reason, in the case where the two bead model has the cyclic structure, the energy depending on the distance between the main chain particles at both ends is calculated every time the (i+1)th main chain particle is moved (the above energy is not excluded from the calculation targets when the two bead model has the cyclic structure).

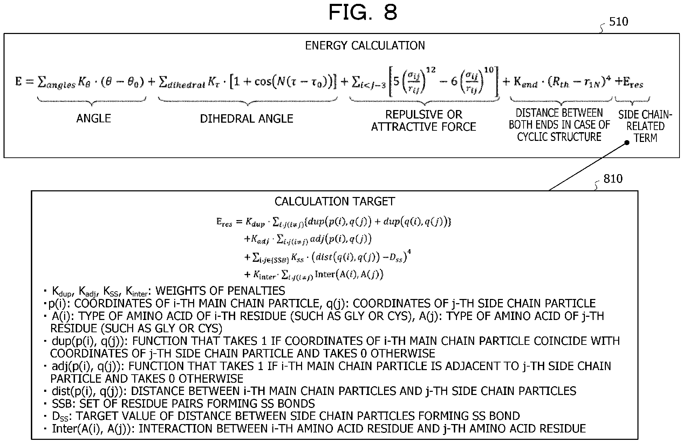

As indicated with reference sign 810 in FIG. 8, the stable conformation search system according to the first embodiment performs the process of calculating the side chain particle-related energy in accordance with Formula 2 presented below, while imposing:

- a penalty in a case where the coordinates of an i-th side chain particle coincide with the coordinates of any of the main chain particles other than the i-th main chain particle or any of the side chain particles other than the i-th side chain particle;
- a penalty in a case where the i-th side chain particle is located adjacent to a j-th main chain particle ($i \neq j$); and
- a penalty in a case where side chain particles forming an SS bond are separated from each other (a penalty in a case where the side chain particles are not adjacent to each other or a penalty in a case where a distance between the side chain particles forming the SS bond is equal to or greater than a target value).

$$E_{res} = K_{dup} \cdot \Sigma_{i,j(i \neq j)}\{dup(p(i), q(j)) + dup(q(i), q(j))\}$$
$$+ K_{adj} \cdot \Sigma_{i,j(i \neq j)} \, adj(p(i), q(j))$$
$$+ \Sigma_{i,j \in \{SSB\}} K_{ss} \cdot \left(dist(q(i), q(j)) - D_{ss}\right)^4$$
$$+ K_{inter} \cdot \Sigma_{i,j(i \neq j)} Inter(A(i), A(j))$$

... (Formula 2)

In Formula 2 above, $K_{dup}$, $K_{adj}$, $K_{ss}$, and $K_{inter}$ denote weights of the respective penalties. In Formula 2 above: p(i) denotes the coordinates of an i-th main chain particle; q(j) denotes the coordinates of a j-th side chain particle; dup((p(i), q(j)) is a function that takes "1" if the coordinates of the i-th main chain particle and the coordinates of the j-th side chain particle coincide with each other or takes "0" otherwise; adj(p(i), q(j)) is a function that takes "1" if the coordinates of the i-th main chain particle and the coordinates of the j-th side chain particle are adjacent to each other or takes "0" otherwise; dist(q(i), q(j)) denotes a distance between the i-th side chain particle and the j-th side chain particle; SSB denotes a set of side chain particles forming SS bonds; Dss denotes a target value of a distance between the side chain particles forming each SS bond; and Inter (A(i), A(j)) denotes an energy based on an interaction between an i-th amino acid residue and a j-th amino acid residue.

Since the i-th amino acid residue and the j-th amino acid residue generate an energy in between depending on the repulsive force or attractive force according to the types of the residues, the energy depending on the repulsive force or attractive force according to the types of the residues is added in Formula 2 presented above. Here, A(i) denotes the type of the i-th amino acid residue, and A(j) denotes the type of the j-th amino acid residue.

As described above, in the first embodiment, the energy calculation processing based on the state transitions in consideration of the side chain particles and the energy calculation processing in consideration of the characteristics of the side chain particles are performed.

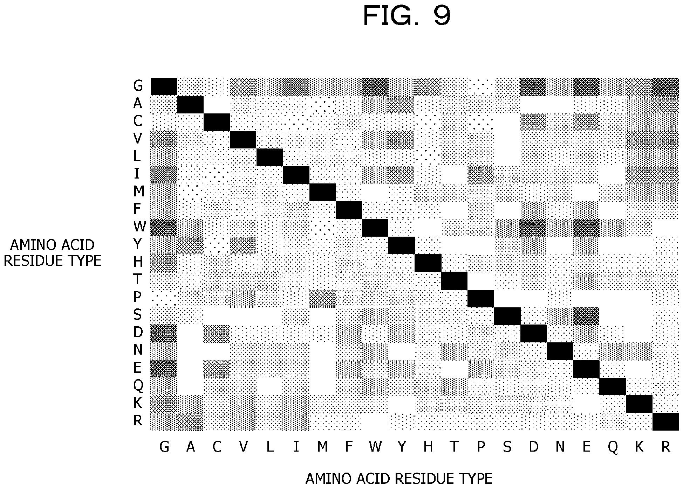

FIG. 9 is a diagram illustrating the magnitude of an interaction between amino acid residues. FIG. 9 has a horizontal axis and a vertical axis in which types (20 types) of amino acid residues are arranged, and a color of each region at which an amino acid residue on the horizontal axis and an amino acid residue on the vertical axis intersect each other represents the magnitude of an interaction between the pair of the types of the amino acid residues. In Formula 2 presented above, the final term, Inter (A(i), A(j)) is derived based on FIG. 9 (for example, Inter (A(i), A(j)) is derived by selecting A(i) from the horizontal axis and selecting A(j) from the vertical axis).

As described above, in the first embodiment, the energy calculation processing in consideration of the types of the amino acid residues is performed.

<SYSTEM CONFIGURATION OF STABLE CONFORMATION SEARCH SYSTEM AND FUNCTIONAL CONFIGURATIONS OF APPARATUSES>

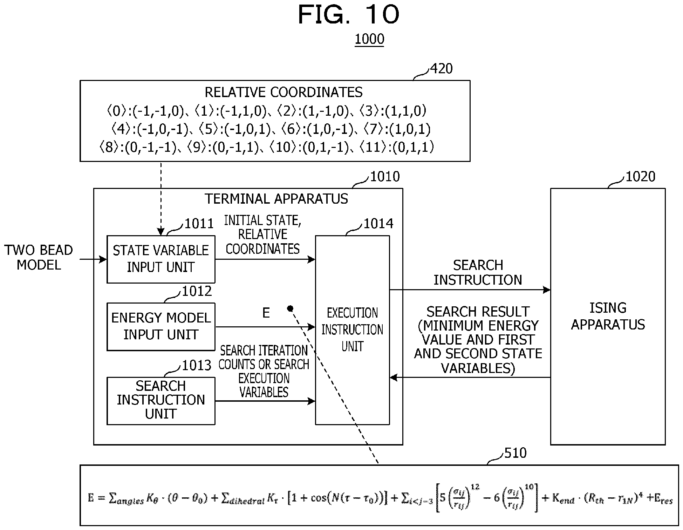

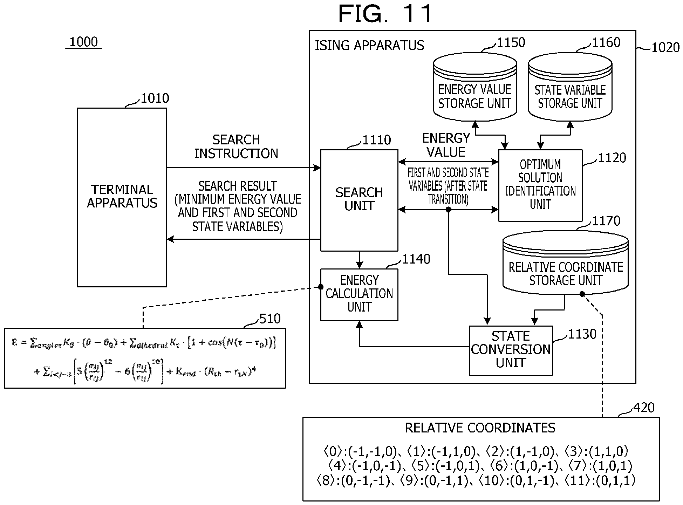

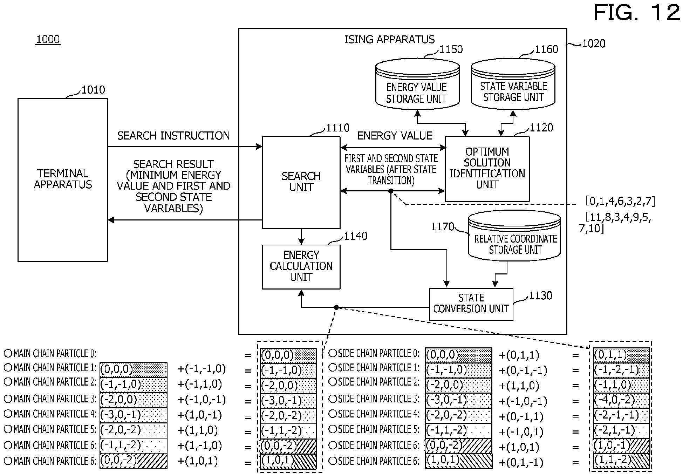

Next, a system configuration of the stable conformation search system, functional configurations of apparatuses, and a specific example of processing according to the first embodiment will be described with reference to FIGs. 10 to 12. FIG. 10 is a diagram illustrating an example of the system configuration of the stable conformation search system and the functional configuration of a terminal apparatus. FIG. 11 is a diagram illustrating an example of the system configuration of the stable conformation search system and the functional configuration of an Ising apparatus. FIG. 12 illustrates a specific example of the processing by the Ising apparatus.

(1) SYSTEM CONFIGURATION OF STABLE CONFORMATION SEARCH SYSTEM AND FUNCTIONAL CONFIGURATION OF TERMINAL APPARATUS

As illustrated in FIG. 10, a stable conformation search system 1000 according to the first embodiment includes a terminal apparatus 1010 and an Ising apparatus 1020.

A stable conformation search instruction program is installed in the terminal apparatus 1010. Executing the program, the terminal apparatus 1010 functions as a state variable input unit 1011, an energy model input unit 1012, a search instruction unit 1013, and an execution instruction unit 1014.

The state variable input unit 1011 receives input of a two bead model in an initial state, which is a stable conformation search target. The state variable input unit 1011 identifies the two bead model by receiving the setting of the number N of amino acid residues (N is an integer of two or more) and an amino acid residue sequence. The state variable input unit 1011 sets an initial state of the two bead model by randomly determining N-1 values in the first state variable and N values in the second state variable.

The state variable input unit 1011 receives input of the relative coordinates (reference sign 420) for representing the first state variable. The execution instruction unit 1014 is notified of the two bead model in the initial state and the relative coordinates (reference sign 420), the input of which is received by the state variable input unit 1011.

The energy model input unit 1012 receives input of the calculation formula (reference sign 510) for use to execute the energy calculation processing. The execution instruction unit 1014 is notified of the calculation formula (reference sign 510), the input of which is received by the energy model input unit 1012.

The search instruction unit 1013 receives input of search iteration counts or search execution variables for use in a search for a stable conformation of the two bead model. The search iteration count or search execution variable mentioned herein is equivalent to the number of updates of each of the state variables in the Ising apparatus 1020. The execution instruction unit 1014 is notified of the search iteration counts or the search execution variables, the input of which is received by the search instruction unit 1013.

The execution instruction unit 1014 instructs the Ising apparatus 1020 to search for a stable conformation based on all the information notified of by the state variable input unit 1011, the energy model input unit 1012, and the search instruction unit 1013. The execution instruction unit 1014 issues the search instruction to the Ising apparatus 1020 by transmitting the following:

- the search target: the two bead model having the number N of the amino acid residues,
- the initial state of the two bead model: the first and second state variables randomly determined,
- the representation methods of the first and second state variables: the relative coordinates (reference sign 420),
- the energy calculation method in the search processing: the calculation formula (reference sign 510), and
- the numbers of updates of the state variables in the search processing: the search iteration counts or the search execution variables.

The execution instruction unit 1014 receives a search result from the Ising apparatus 1020 as a result of issuing the search instruction to the Ising apparatus 1020. The search result received by the execution instruction unit 1014 includes the calculation result of the minimum energy and the first and second state variables achieving the minimum energy. Accordingly, the execution instruction unit 1014 is enabled to output the stable conformation of the two bead model and the energy based on the interactions between the main chain particles, the interactions between the main chain particles and the side chain particles, and the like in the two bead model in the stable conformation.

## (2) SYSTEM CONFIGURATION OF STABLE CONFORMATION SEARCH SYSTEM AND FUNCTIONAL CONFIGURATION OF ISING APPARATUS

The functional configuration of the Ising apparatus 1020 will be described with reference to FIG. 11 (since the system configuration of the stable conformation search system 1000 is described above with reference to FIG. 10, and the description thereof will be omitted).

A stable conformation search program is installed in the Ising apparatus 1020. Executing the program, the Ising apparatus 1020 functions as a search unit 1110, an optimum solution identification unit 1120, a state conversion unit 1130, and an energy calculation unit 1140.

The search unit 1110 searches for a stable conformation of the two bead model based on a search instruction from the terminal apparatus 1010, and transmits a search result to the terminal apparatus 1010. For example, for the two bead model, the search unit 1110 updates (changes) the first and second state variables within ranges of the instructed search iteration counts (or search execution variables). For every state transition of the two bead model made by updating the first and second state variables, the search unit 1110 determines whether or not the transition is acceptable based on the energy values calculated. The search unit 1110 transmits, as the search result to the terminal apparatus 1010, the minimum energy value identified among the energy values for each of which the transition is determined as acceptable, and the first and second state variables achieving the minimum energy value.

The optimum solution identification unit 1120 is an example of an identification unit. Every time the optimum solution identification unit 1120 is notified of the energy value by the search unit 1110, the optimum solution identification unit 1120 compares the notified energy value with the energy value stored in an energy value storage unit 1150, and stores the notified energy value in the energy value storage unit 1150 when the notified energy value is smaller than the stored energy value. In this way, the optimum solution identification unit 1120 is able to identify the minimum energy value.

Every time the optimum solution identification unit 1120 is notified of the updated first and second state variables by the search unit 1110, the optimum solution identification unit 1120 holds the updated first and second state variables. In a case where the optimum solution identification unit 1120 is notified of the corresponding energy value by the search unit 1110 and stores the corresponding energy value in the energy value storage unit 1150, the optimum solution identification unit 1120 stores the held first and second state variables in a state variable storage unit 1160. In this way, the optimum solution identification unit 1120 is able to identify the first and second state variables corresponding to the minimum energy value.

The state conversion unit 1130 is an example of first and second calculation units. Every time the state conversion unit 1130 is notified of the updated first and second state variables by the search unit 1110, the state conversion unit 1130 refers to a relative coordinate storage unit 1170 and calculates the coordinates of each main chain particle and each side chain particle in the two bead model after the state transition. Note that the relative coordinate storage unit 1170 stores in advance the relative coordinates transmitted together with the search instruction from the terminal apparatus 1010.

The energy calculation unit 1140 is notified of the coordinates of each main chain particle and each side chain particle in the two bead model after the state transition, which are calculated by the state conversion unit 1130.

The energy calculation unit 1140 is an example of a calculation unit. Every time the energy calculation unit 1140 is notified of the coordinates of each main chain particle and each side chain particle in the two bead model after the state transition by the state conversion unit 1130, the energy calculation unit 1140 executes the energy calculation processing. For example, the energy calculation unit 1140 calculates the energy value concerning the calculation targets described with reference to FIGs. 6 to 8 based on the calculation formula (reference sign 510) transmitted together with the search instruction from the terminal apparatus 1010. The search unit 1110 is notified of the energy value calculated by the energy calculation unit 1140, and performs the next search by treating the updated first and second state variables as the first and second state variables before the next update.

(3) SPECIFIC EXAMPLE OF PROCESSING BY ISING APPARATUS

Subsequently, a specific example of the processing by the Ising apparatus 1020 will be described with reference to FIG. 12. FIG. 12 illustrates an example in which the number N of amino acid residues is 8, and the search unit 1110 updates the first state variable to [0, 1, 4, 6, 3, 2, 7], and notifies the optimum solution identification unit 1120 and the state conversion unit 1130 of the updated first state variable. FIG. 12 illustrates the example in which the search unit 1110 updates the second state variable to [11, 8, 3, 4, 9, 5, 7, 10] and notifies the optimum solution identification unit 1120 and the state conversion unit 1130 of the updated second state variable.

As illustrated in FIG. 12, in response to the notification of the updated first and second state variables from the search unit 1110, the state conversion unit 1130 calculates the coordinates of each of the eight main chain particles and the coordinates of each of the eight side chain particles based on the relative coordinates as follows:

- the coordinates of the zeroth main chain particle = (0, 0, 0),

- the coordinates of the first main chain particle = (0, 0, 0) + (-1, -1, 0) = (-1, -1, 0),

- the coordinates of the second main chain particle = (-1, -1, 0) + (-1, 1, 0) = (-2, 0, 0),

- the coordinates of the third main chain particle = (-2, 0, 0) + (-1, 0, -1) = (-3, 0, -1),

- the coordinates of the fourth main chain particle = (-3, 0, -1) + (1, 0, -1) = (-2, 0, -2),

- the coordinates of the fifth main chain particle = (-2, 0, -2) + (1, 1, 0) = (-1, 1, -2),

- the coordinates of the sixth main chain particle = (-1, 1, -2) + (1, -1, 0) = (0, 0, -2),

- the coordinates of the seventh main chain particle = (0, 0, -2) + (1, 0, 1) = (1, 0, -1),

- the coordinates of the zeroth side chain particle = (0, 0, 0) + (0, 1, 1) = (0, 1, 1),

- the coordinates of the first side chain particle = (-1, -1, 0) + (0, -1, -1) = (-1, -2, -1),

- the coordinates of the second side chain particle = (-2, 0, 0) + (1, 1, 0) = (-1, 1, 0),

- the coordinates of the third side chain particle = (-3, 0, -1) + (-1, 0, -1) = (-4, 0, -2),

- the coordinates of the fourth side chain particle = (-2, 0, -2) + (0, -1, 1) = (-2, -1, -1),

- the coordinates of the fifth side chain particle = (-1, 1, -2) + (-1, 0, 1) = (-2, 1, -1),

- the coordinates of the sixth side chain particle = (0, 0, -2) + (1, 0, 1) = (1, 0, -1), and

- the coordinates of the seventh side chain particle = (1, 0, -1) + (0, 1, -1) = (1, 1, -2).

<HARDWARE CONFIGURATION OF TERMINAL APPARATUS>

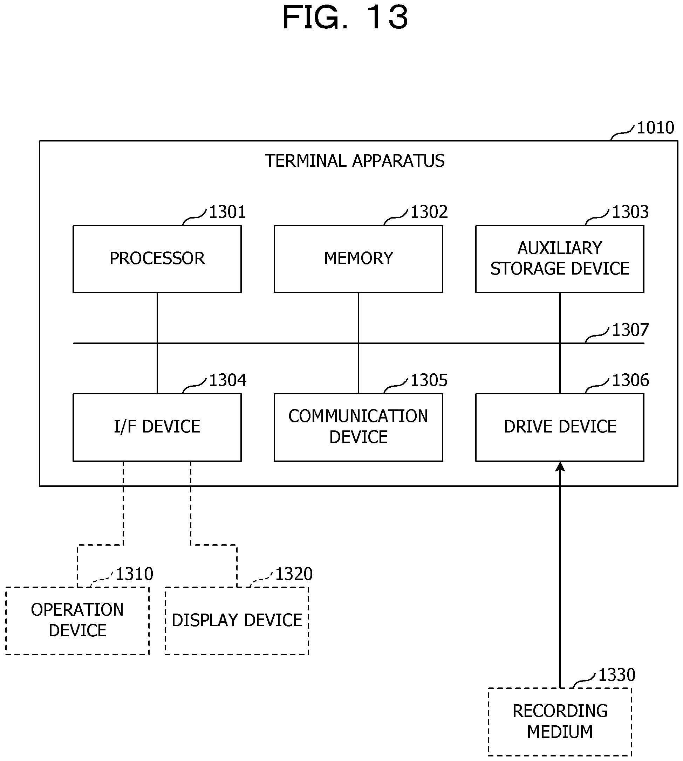

Next, a hardware configuration of the terminal apparatus 1010 will be described. FIG. 13 is a diagram illustrating an example of the hardware configuration of the terminal apparatus.

As illustrated in FIG. 13, the terminal apparatus 1010 includes a processor 1301, a memory 1302, an auxiliary storage device 1303, an interface (I/F) device 1304, a communication device 1305, and a drive device 1306. In the terminal apparatus 1010, the processor 1301, the memory 1302, the auxiliary storage device 1303, the I/F device 1304, the communication device 1305, and the drive device 1306 are coupled to each other via a bus 1307.

The processor 1301 includes various computing devices such as a central processing unit (CPU) and a graphics processing unit (GPU). The processor 1301 loads various programs (for example, the stable conformation search instruction program and so on) onto the memory 1302 and executes the programs.

The memory 1302 includes main storage devices such as a read-only memory (ROM) and a random-access memory (RAM). The processor 1301 and the memory 1302 form a so-called computer. The computer implements the above-described various functions when the processor 1301 executes the various programs loaded onto the memory 1302.

The auxiliary storage device 1303 stores the various programs and various types of information to be used in execution of the various programs by the processor 1301.

The I/F device 1304 is a coupling device through which the terminal apparatus 1010 is coupled to an operation device 1310 and an output device 1320, which are examples of external devices.

The communication device 1305 is a communication device for communicating with the Ising apparatus 1020 via a network.

The drive device 1306 is a device where to set a recording medium 1330. The recording medium 1330 mentioned herein includes a medium that records information optically, electrically, or magnetically such as a CD-ROM, a flexible disk, or a magneto-optical disk. The recording medium 1330 may also include a semiconductor memory that records information electrically such as a ROM or a flash memory, and the like.

As for the various programs to be installed in the auxiliary storage device 1303, the distributed recording medium 1330 set in the drive device 1306 and the various programs recorded in the recording medium 1330 are read and then installed therein. Alternatively, the various programs to be installed in the auxiliary storage device 1303 may be installed by being downloaded from the network via the communication device 1305.

Although only the hardware configuration of the terminal apparatus 1010 is described and the hardware configuration of the Ising apparatus 1020 is not described herein, the hardware configuration of the Ising apparatus 1020 may be the same as or similar to that of, for example, the terminal apparatus 1010. Alternatively, the hardware configuration of the Ising apparatus 1020 may be the same as or similar to the hardware configuration of a so-called quantum computer.

<SEQUENCE OF STABLE CONFORMATION SEARCH PROCESSING>

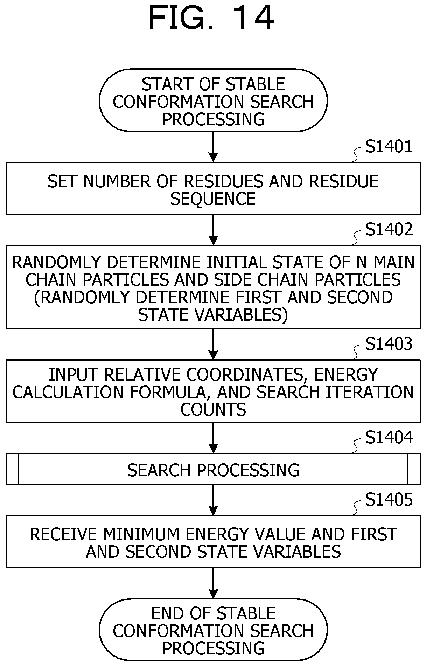

Next, a sequence of stable conformation search processing performed by the stable conformation search system 1000 will be described. FIG. 14 is a flowchart illustrating the sequence of the stable conformation search processing.

At step S1401, the terminal apparatus 1010 receives the setting of the number N of amino acid residues (N is an integer of two or more) and an amino acid residue sequence and thereby identifies a two bead model (including N main chain particles and N side chain particles) as a stable conformation search target.

At step S1402, the terminal apparatus 1010 randomly determines an initial state of the two bead model including the N main chain particles and the N side chain particles. For example, the terminal apparatus 1010 randomly determines N-1 values in the first state variable and N values in the second state variable.

At step S1403, the terminal apparatus 1010 receives input of the relative coordinates for representing the first and second state variables. The terminal apparatus 1010 receives input of the calculation formula for used in the energy calculation processing in the search processing. The terminal apparatus 1010 receives the search iteration counts or the search execution variables as the numbers of updates of the first and second state variables in the search processing. The terminal apparatus 1010 transmits a search instruction based on all the received information to the Ising apparatus 1020 to search for the stable conformation of the two bead model.

At step S1404, the Ising apparatus 1020 executes the search processing. Details of the search processing by the Ising apparatus 1020 will be described later.

At step S1405, the terminal apparatus 1010 receives a search result from the Ising apparatus 1020, and outputs a calculation result of the minimum energy value for the two bead model as the search target and the first and second state variables achieving the minimum energy value.

<SEQUENCE OF SEARCH PROCESSING>

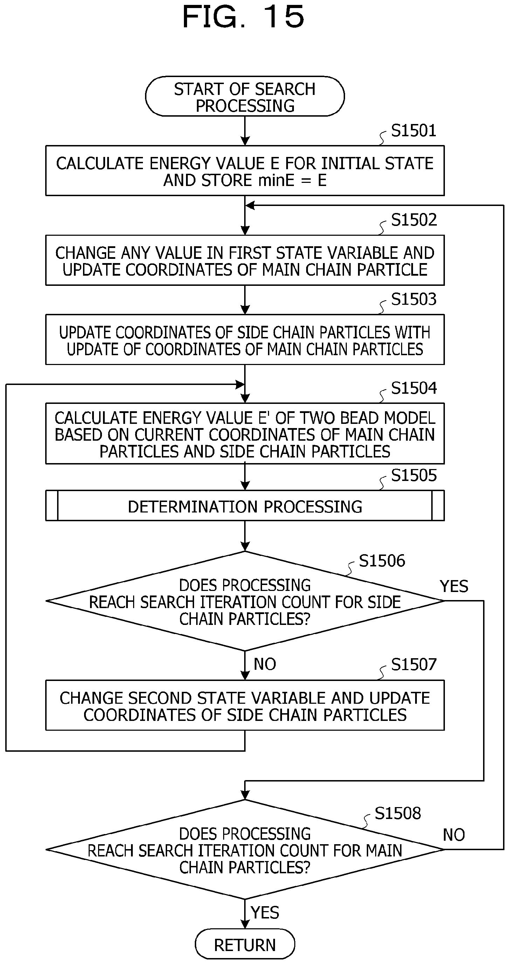

The details of the search processing (step S1404) by the Ising apparatus 1020 will be described with reference to FIG. 15. FIG. 15 is a flowchart illustrating the details of the search processing.

At step S1501 in FIG. 15, the Ising apparatus 1020 calculates an energy value for the two bead model in the initial state, and stores the calculated energy value as the minimum energy value (minE) at the current time point.

At step S1502, the Ising apparatus 1020 randomly changes any one of the N-1 values in the first state variable specifying the coordinates of the N main chain particles in the two bead model and thereby updates the coordinates of the main chain particles. It is noted that the first state variable specifying the coordinates of the N main chain particles is represented by the relative coordinates. For example, processing at the step S1502, such as the randomly changing of the first state variable and the updating the coordinates of the main chain particles, corresponds to processing of calculating coordinates of an (i+1)th main chain particle in the sequence in the lattice space, by using coordinates of an i-th main chain particle in the sequence in the lattice space and a first state variable represented by relative coordinates between the i-th main chain particle and the (i+1)th main chain particle in the lattice space.

At step S1503, the Ising apparatus 1020 updates the coordinates of the side chain particles with the update of the coordinates of the main chain particles as described in claim 1. It is noted that the coordinates of the side chain particles are represented by the relative coordinates. For example, processing at the step S1503, such as the updating of the coordinates of the side chain particles, corresponds to processing of calculating coordinates of an (i+1)th side chain particle corresponding to the (i+1)th main chain particle in the lattice space, by using the coordinates of the (i+1)th main chain particle in the lattice space and a second state variable represented by relative coordinates between the (i+1)th main chain particle and the (i+1)th side chain particle.

At step S1504, the Ising apparatus 1020 calculates an energy value E' of the two bead model based on the current coordinates of the main chain particles and the side chain particles.

At step S1505, the Ising apparatus 1020 performs determination processing of determining whether or not the calculated energy value E' is the minimum energy value. Details of the determination processing (step S1505) will be described later.

At step S1506, the Ising apparatus 1020 determines whether or not the processing reaches the search iteration count for the side chain particles. If determining at step S1506 that the processing does not reach the search iteration count for the side chain particles (NO at step S1506), the Ising apparatus 1020 proceeds to step S1507.

The Ising apparatus 1020 changes the second state variable and updates the coordinates of the side chain particles at step S1507, and then returns to step S1504.

On the other hand, if determining at step S1506 that the processing reaches the search iteration count (YES at step S1506), the Ising apparatus 1020 resets the search iteration count for the side chain particles and then proceeds to step S1508.

At step S1508, the Ising apparatus 1020 determines whether or not the processing reaches the search iteration count for the main chain particles. If determining at S1508 that the processing does not reach the search iteration count for the main chain particles (NO at step S1508), the Ising apparatus 1020 returns to step S1502.

On the other hand, if determining at S1508 that the processing reaches the search iteration count for the main chain particles (YES at step S1508), the Ising apparatus 1020 terminates the search processing and returns to step S1405 in FIG. 14.

<SEQUENCE OF DETERMINATION PROCESSING>

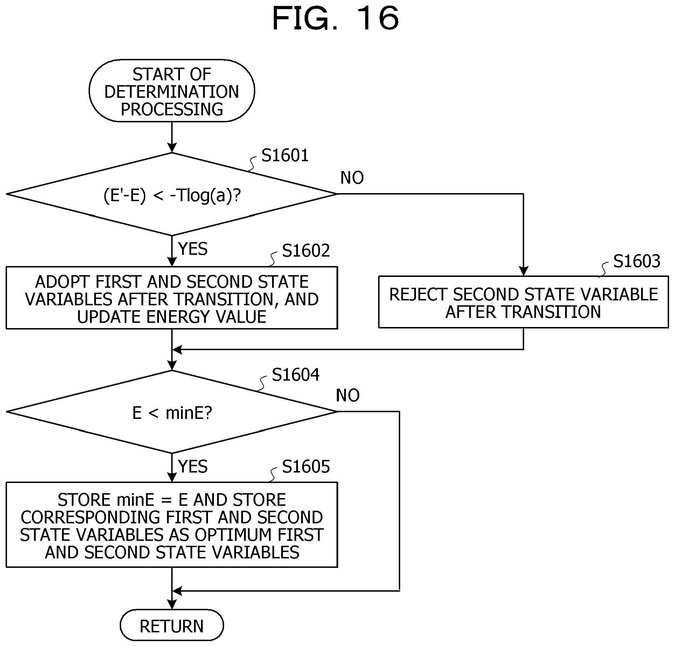

The details of the determination processing (step S1505) by the Ising apparatus 1020 will be described with reference to FIG. 16. FIG. 16 is a flowchart illustrating the details of the determination processing.

At step S1601 in FIG. 16, the Ising apparatus 1020 calculates a difference between an energy value E stored as the minimum energy value (minE) at the current time point and the energy value E' calculated at step S1504. The Ising apparatus 1020 determines whether or not the calculated difference is smaller than a predetermined thermal noise (-Tlog(a) in an example of FIG. 16). Here, a is a uniform random number and is a value satisfying $a \in [0, 1]$.

If determining at step S1601 that the calculated difference is smaller than the predetermined thermal noise (-Tlog(a)) (YES at step S1601), the Ising apparatus 1020 proceeds to step S1602.

At step S1602, the Ising apparatus 1020 determines that the transition is acceptable, adopts the updated first or second state variable, and updates the energy value (replaces the energy value E with the energy value E').

On the other hand, if determining at step S1601 that the calculated difference is equal to or greater than the predetermined thermal noise (-Tlog(a)) (NO at step S1601), the Ising apparatus 1020 proceeds to step S1603.

At step S1603, the Ising apparatus 1020 determines that the transition is unacceptable and discards the updated first or second state variable.

At step S1604, the Ising apparatus 1020 determines whether or not the energy value E is smaller than the minimum energy value (minE). If determining at step S1604 that the energy value E is smaller than the minimum energy value (minE) (YES at step S1604), the Ising apparatus 1020 proceeds to step S1605.

At step S1605, the Ising apparatus 1020 stores the energy value E as the minimum energy value (minE) at the current time point. The Ising apparatus 1020 stores the first and second state variables as the first and second state variables corresponding to the minimum energy value at the current time point.

On the other hand, if determining at step S1604 that the energy value E is equal to or greater than the minimum energy value (minE) (NO at step S1604), the Ising apparatus 1020 terminates the determination processing and returns to step S1506 in FIG. 15.

<EFFECT OF REDUCING AMOUNT OF CALCULATION>

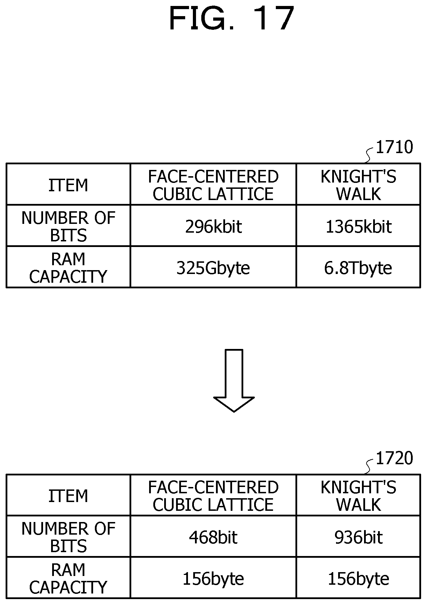

An effect of reducing the amount of calculation in the stable conformation search processing by the stable conformation search system 1000 according to the first embodiment will be described next. FIG. 17 is a diagram illustrating an example of the effect of reducing the amount of calculation.

In FIG. 17, reference sign 1710 indicates, as a comparative example, amounts of calculation when a search for a stable conformation in the case where the number N of amino acid residues is 20, is performed by: [a] using first and second state variables represented by absolute coordinates and, [b] calculating the energy without limiting calculation targets.

On the other hand, reference sign 1720 indicates amounts of calculation when a search for a stable stricture in the case where the number N of amino acid residues is 20, is performed by the stable conformation search system 1000 according to the first embodiment, by [a] using the first and second state variables represented by the relative coordinates and [b] limiting the calculation targets to the energy differences generated with movement of the position of each of the main chain particles and the side chain particles.

In items for the amounts of calculation presented with reference signs 1710 and 1720, "NUMBER OF BITS" represents the number of bits used to express the coordinates of the main chain particles and the side chain particles. "RAM CAPACITY" represents the capacity of a memory used for the energy calculation processing.

In each of the items, the amounts of calculation in the respective cases where the lattice space is a face-centered cubic lattice and a knight's walk lattice are presented.

As is apparent from the comparison between reference signs 1710 and 1720, the stable conformation search system 1000 according to the first embodiment makes it possible to significantly reduce the amounts of calculation in the stable conformation search processing.

For example, the stable conformation search system 1000 according to the first embodiment searches for the stable conformation by [a] using the first and second state variables represented by the relative coordinates and [b] limiting the calculation target to the energy difference before and after each state transition, and thereby achieves a reduction in the amount of calculation as compared with the case where the stable conformation is searched for by using the absolute coordinates and without limiting the calculation target.

For example, the stable conformation search system 1000 according to the first embodiment makes it possible to significantly increase the number N of amino acid residues in the two bead model as the search target under the current computing capacity.

As is clear from the above description, the stable conformation search system 1000 according to the first embodiment obtains a two bead model in which N main chain particles arranged in a sequence and N side chain particles correspondingly linked to the respective N main chain particles are arranged in a lattice space, and calculates the coordinates of an (i+1)th main chain particle in the sequence in the lattice space by using:

- the coordinates of the i-th main chain particle in the sequence in the lattice space; and
- the first state variable represented by the relative coordinates between the i-th main chain particle and the (i+1)th main chain particle in the lattice space.

The stable conformation search system 1000 according to the first embodiment obtains a two bead model in which N main chain particles arranged in a sequence and N side chain particles correspondingly boned to the respective N main chain particles are arranged in a lattice space, and calculates the coordinates of an (i+1)th side chain particle in the sequence in the lattice space by using:

- the coordinates of the (i+1)th main chain particle in the sequence in the lattice space; and
- the second state variable represented by the relative coordinates between the (i+1)th main chain particle and the (i+1)th side chain particle in the lattice space.

Every time any of the first state variable and the second state variable is changed, the stable conformation search system 1000 according to the first embodiment repeatedly executes the processing of calculating the energy value of the two bead model.

The stable conformation search system 1000 identifies the first state variable and the second state variable with which the energy value is the local minimum value.

As described above, the stable conformation search system 1000 according to the first embodiment uses the two bead model to search for a stable conformation in consideration of the side chain particles. Accordingly, the stable conformation search system 1000 according to the first embodiment makes it possible to improve the search accuracy as compared with a case where a stable conformation of main chain particles is searched for by using a one bead model.

[Second Embodiment]

Although the above first embodiment describes the case where any one of the values included in the first and second state variables in the search processing is randomly selected and updated, the value may be selected by any method other than the random selection. For example, the value may be selected and updated in a predetermined order such as an order from the first value to the (N-1)th value among the (N-1) values included in the first state variable, or an order from the first value to the N-th value among the N values included in the second state variable.

In the case of a face-centered cubic lattice, there are 12 values to which a value in each of the first and second state variables may be updated, and to which value the value in the first or second state variable is to be updated may be randomly determined.

The weights of the terms such as $K_\theta$, $K_\tau$, and $K_{end}$ are fixed in the calculation formula (reference sign 510) used for the energy calculation processing in the first embodiment described above, but the weights of the terms may be changed depending on types of amino acids, for example.

The first embodiment is described above in the case where, every time the first state variable for the main chain particles is changed, the local minimum value is searched for by calculating the energy of the two bead model concerning various values in the second state variable for the side chain particles. However, the method of searching for the local minimum value is not limited to this. For example, the minimum value may be searched for by calculating the energy of the two bead model concerning various values in the first state variable for the main chain particles. After all the values in the first state variable for the main chain particles are fixed, the local minimum value may be searched for by calculating the energy of the two bead model concerning various values in the second state variable for the side chain particles.

In the above first embodiment, the face-centered cubic lattice and the knight's walk lattice are exemplified as the lattice space, but the lattice space is not limited to these.

The first embodiment is described above such that the stable conformation search system 1000 includes the terminal apparatus 1010 and the Ising apparatus 1020, but the terminal apparatus 1010 and the Ising apparatus 1020 may be included in an integrated apparatus. In this case, the stable conformation search program includes the stable conformation search instruction program, and the Ising apparatus 1020 implements both of the functional units of the terminal apparatus 1010 described with reference to FIG. 10 and the functional units of the Ising apparatus 1020 described with reference to FIG. 11.

Modes as in appendixes to be described below are conceivable according to the disclosed technique.

The present disclosure is not limited to the configurations illustrated herein but may include a configuration such as a combination of any of the configurations and the like exemplified in the above embodiments with other elements, and the like. These aspects may be changed without departing from the gist of the present disclosure and appropriately determined in accordance with application modes thereof.

## Claims

1. A stable conformation search system comprising:

   a first calculation unit configured to
   obtain a model in which N (N is an integer of two or more) main chain particles arranged in a sequence and N side chain particles correspondingly linked to the respective N main chain particles are arranged in a lattice space, and calculate coordinates of an (i+1)th main chain particle in the sequence in the lattice space, by using coordinates of an i-th main chain particle in the sequence in the lattice space and a first state variable represented by relative coordinates between the i-th main chain particle and the (i+1)th main chain particle in the lattice space;
   a second calculation unit configured to calculate coordinates of an (i+1)th side chain particle corresponding to the (i+1)th main chain particle in the lattice space, by using the coordinates of the (i+1)th main chain particle in the lattice space and a second state variable represented by relative coordinates between the (i+1)th main chain particle and the (i+1)th side chain particle;
   a calculation unit configured to calculate a value of an energy of the model in a case where any one of the first state variable and the second state variable is changed; and
   an identification unit configured to identify the first state variable and the second state variable with which the value of the energy is a local minimum value.

2. The stable conformation search system according to claim 1, further comprising:
   a search unit configured to change any one of (N-1) values included in the first state variable and any one of N values included in the second state variable.

3. The stable conformation search system according to claim 1, wherein
   a position of the (i+1)th side chain particle in the lattice space is determined so as not to coincide with positions of the main chain particles and the side chain particles other than the (i+1)th side chain particle.

4. The stable conformation search system according to claim 1, wherein
   the calculation unit is configured to calculate, based on the coordinates of each of the main chain particle and the side chain particle in the lattice space, the value of the energy corresponding to an interaction between the main chain particles and the side chain particles.

5. The stable conformation search system according to claim 4, wherein
the energy corresponding to the interaction includes any one of: an energy depending on an angle among the main chain particles, an energy depending on a dihedral angle among the main chain particles, an energy depending on a repulsive force or attractive force between the main chain particles, an energy depending on a distance between the main chain particles at both ends in a case where the model has a cyclic structure, or an energy depending on characteristics of the side chain particles.

6. The stable conformation search system according to claim 5, wherein
the energy depending on the characteristics of the side chain particles includes any one of:

a penalty in a case where the coordinates of the (i+1)th side chain particle in the lattice space coincide with the coordinates of any of the main chain particles other than the (i+1)th main chain particle and the side chain particles in the lattice space,
a penalty in a case where the coordinates of the (i+1)th side chain particle in the lattice space are adjacent to the coordinates of any of the main chain particles other than the (i+1)th main chain particle in the lattice space,
a penalty in a case where a distance between the (i+1)th side chain particle and the side chain particle forming an SS bond with the (i+1)th side chain particle is equal to or greater than a target value, or
an energy derived according to a pair of types of amino acids.

7. The stable conformation search system according to claim 2, wherein
the calculation unit is configured to calculate a difference between the energy after the first and second state variables are updated and the energy before the first and second state variables are updated.

8. The stable conformation search system according to claim 7, wherein
the search unit is configured to determine that a change in the first and second state variables is acceptable in a case where the difference calculated by the calculation unit satisfies a predetermined condition.

9. A stable conformation search method implemented by a computer, the stable conformation search method comprising:

obtaining a model in which N (N is an integer of two or more) main chain particles arranged in a sequence and N side chain particles correspondingly linked to the respective N main chain particles are arranged in a lattice space;
calculating coordinates of an (i+1)th main chain particle in the sequence in the lattice space, by using coordinates of an i-th main chain particle in the sequence in the lattice space and a first state variable represented by relative coordinates between the i-th main chain particle and the (i+1)th main chain particle in the lattice space;
calculating coordinates of an (i+1)th side chain particle corresponding to the (i+1)th main chain particle in the lattice space by using the coordinates of the (i+1)th main chain particle in the lattice space and a second state variable represented by relative coordinates between the (i+1)th main chain particle and the (i+1)th side chain particle;
calculating a value of an energy of the model in a case where any one of the first state variable and the second state variable is changed; and
identifying the first state variable and the second state variable with which the value of the energy is a local minimum value.

10. A stable conformation search program comprising instructions which, when executed by a computer, cause the computer to perform processing comprising:

obtaining a model in which N (N is an integer of two or more) main chain particles arranged in a sequence and N side chain particles correspondingly linked to the respective N main chain particles are arranged in a lattice space;
calculating coordinates of an (i+1)th main chain particle in the sequence in the lattice space, by using coordinates of an i-th main chain particle in the sequence in the lattice space and a first state variable represented by relative coordinates between the i-th main chain particle and the (i+1)th main chain particle in the lattice space;
calculating coordinates of an (i+1)th side chain particle corresponding to the (i+1)th main chain particle in the lattice space by using the coordinates of the (i+1)th main chain particle in the lattice space and a second state variable represented by relative coordinates between the (i+1)th main chain particle and the (i+1)th side chain particle;

calculating a value of an energy of the model in a case where any one of the first state variable and the second state variable is changed; and

identifying the first state variable and the second state variable with which the value of the energy is a local minimum value.

FIG. 1

MIDDLE MOLECULE DRUG CANDIDATES

NARROW DOWN AND COARSE-GRAIN

TARGET PROTEIN

STABLE CONFORMATION SEARCH

TWO BEAD MODEL IN INITIAL STATE

TWO BEAD MODEL OF STABLE CONFORMATION

STABLE CONFORMATION SEARCH

ALL-ATOM MODEL OF STABLE CONFORMATION

DRUG EFFICACY VERIFICATION

WET EXPERIMENT

# FIG. 2

STABLE
CONFORMATION
SEARCH

TWO BEAD
MODEL IN
INITIAL
STATE

TWO BEAD
MODEL OF STABLE
CONFORMATION

SEARCH FOR TWO BEAD MODEL OF STABLE CONFORMATION BY
IDENTIFYING MINIMUM ENERGY VALUE
→ USE MARKOV CHAIN MONTE CARLO METHOD AND PARALLEL
TEMPERING METHOD

+

- CALCULATE COORDINATES OF (i+1)th MAIN CHAIN PARTICLE BY USING FIRST STATE VARIABLE REPRESENTED
  BY RELATIVE COORDINATES FROM i-TH MAIN CHAIN PARTICLE
- CALCULATE COORDINATES OF (i+1)th SIDE CHAIN PARTICLE BY USING SECOND STATE VARIABLE REPRESENTED
  BY RELATIVE COORDINATES FROM (i+1)th MAIN CHAIN PARTICLE
- CALCULATE ENERGY BY USING FIRST STATE VARIABLE AND SECOND STATE VARIABLE

FIG. 3A

CANDIDATE POSITION FOR 2ND MAIN CHAIN PARTICLE

CANDIDATE POSITION FOR 3RD MAIN CHAIN PARTICLE

CANDIDATE POSITION FOR 4TH MAIN CHAIN PARTICLE

FIG. 3B

CANDIDATE POSITION FOR 1ST SIDE CHAIN PARTICLE (POSITION OF 2ND MAIN CHAIN PARTICLE IS EXCLUDED)

CANDIDATE POSITION FOR 2ND SIDE CHAIN PARTICLE (POSITIONS OF 1ST AND 3RD MAIN CHAIN PARTICLES ARE EXCLUDED)

CANDIDATE POSITION FOR 3RD SIDE CHAIN PARTICLE (POSITIONS OF 2ND AND 4TH MAIN CHAIN PARTICLES ARE EXCLUDED)

# FIG. 4A

410

<5>   <9>   <11>   <7>

<1>

<0>

<4>   <8>   <10>   <6>

<3>

<2>

420

**RELATIVE COORDINATES**

⟨0⟩:(-1,-1,0)、⟨1⟩:(-1,1,0)、⟨2⟩:(1,-1,0)、⟨3⟩:(1,1,0)
⟨4⟩:(-1,0,-1)、⟨5⟩:(-1,0,1)、⟨6⟩:(1,0,-1)、⟨7⟩:(1,0,1)
⟨8⟩:(0,-1,-1)、⟨9⟩:(0,-1,1)、⟨10⟩:(0,1,-1)、⟨11⟩:(0,1,1)

(a)

FIRST STATE VARIABLE = [0, 1, 4, 6, 3, 2, 7]

⇩

○MAIN CHAIN PARTICLE 0 :                              = (0,0,0)
○MAIN CHAIN PARTICLE 1 :(0,0,0)        + (-1,-1,0)   = (-1,-1,0)
○MAIN CHAIN PARTICLE 2 :(-1,-1,0)      + (-1,1,0)    = (-2,0,0)
○MAIN CHAIN PARTICLE 3 :(-2,0,0)       + (-1,0,-1)   = (-3,0,-1)
○MAIN CHAIN PARTICLE 4 :(-3,0,-1)      + (1,0,-1)    = (-2,0,-2)
○MAIN CHAIN PARTICLE 5 :(-2,0,-2)      + (1,1,0)     = (-1,1,-2)
○MAIN CHAIN PARTICLE 6 :(-1,1,-2)      + (1,-1,0)    = (0,0,-2)
○MAIN CHAIN PARTICLE 7 :(0,0,-2)       + (1,0,1)     = (1,0,-1)

(b)

# FIG. 4B

410

<5>  <9>  <11>  <7>

<1>

<0>

<3>

<2>

<4>  <8>  <10>  <6>

420

RELATIVE COORDINATES

⟨0⟩:(-1,-1,0)、⟨1⟩:(-1,1,0)、⟨2⟩:(1,-1,0)、⟨3⟩:(1,1,0)
⟨4⟩:(-1,0,-1)、⟨5⟩:(-1,0,1)、⟨6⟩:(1,0,-1)、⟨7⟩:(1,0,1)
⟨8⟩:(0,-1,-1)、⟨9⟩:(0,-1,1)、⟨10⟩:(0,1,-1)、⟨11⟩:(0,1,1)

(a)

SECOND STATE VARIABLE = [11, 8, 3, 4, 9, 5, 7, 10]

⇩

○SIDE CHAIN PARTICLE 0 :(0,0,0)    + (0,1,1)    = (0,1,1)
○SIDE CHAIN PARTICLE 1 :(-1,-1,0)    + (0,-1,-1)    = (-1,-2,-1)
○SIDE CHAIN PARTICLE 2 :(-2,0,0)    + (1,1,0)    = (-1,1,0)
○SIDE CHAIN PARTICLE 3 :(-3,0,-1)    + (-1,0,-1)    = (-4,0,-2)
○SIDE CHAIN PARTICLE 4 :(-2,0,-2)    + (0,-1,1)    = (-2,-1,-1)
○SIDE CHAIN PARTICLE 5 :(-1,1,-2)    + (-1,0,1)    = (-2,1,-1)
○SIDE CHAIN PARTICLE 6 :(0,0,-2)    + (1,0,1)    = (1,0,-1)
○SIDE CHAIN PARTICLE 7 :(1,0,-1)    + (0,1,-1)    = (1,1,-2)

(b)

FIG. 5

510

| ENERGY CALCULATION |
|---|

$$E = \sum_{angles} K_\theta \cdot (\theta - \theta_0) + \sum_{dihedral} K_\tau \cdot \left[1 + \cos\left(N(\tau - \tau_0)\right)\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4 + E_{res}$$

| ANGLE | DIHEDRAL ANGLE | REPULSIVE OR ATTRACTIVE FORCE | DISTANCE BETWEEN BOTH ENDS IN CASE OF CYCLIC STRUCTURE | SIDE CHAIN-RELATED TERM |

- $K_\theta$, $K_\tau$, $K_{end}$: WEIGHTS OF TERMS
- $\theta$ : ANGLE FORMED BY THREE CONSECUTIVE MAIN CHAIN PARTICLES,
  $\tau$ : DIHEDRAL ANGLE FORMED BY FOUR CONSECUTIVE MAIN CHAIN PARTICLES
- $\theta_0$ TARGET VALUE OF ANGLE, $\tau_0$ TARGET VALUE OF DIHEDRAL ANGLE
- $r_{ij}$: DISTANCE BETWEEN i-TH MAIN CHAIN PARTICLE AND j-TH MAIN CHAIN PARTICLE,
  $\sigma_{ij}$: DISTANCE BETWEEN i-TH MAIN CHAIN PARTICLE AND j-TH MAIN CHAIN PARTICLE IN NATURAL STATE
- $r_{1N}$: DISTANCE BETWEEN MAIN CHAIN PARTICLES AT BOTH ENDS,
  $R_{th}$: TARGET VALUE FOR DISTANCE BETWEEN MAIN CHAIN PARTICLES AT BOTH ENDS

# FIG. 6

510

ENERGY CALCULATION

$$E = \sum_{angles} K_\theta \cdot (\theta - \theta_0) + \sum_{dihedral} K_\tau \cdot \left[1 + \cos\left(N(\tau - \tau_0)\right)\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4 + E_{res}$$

| ANGLE | DIHEDRAL ANGLE | REPULSIVE OR ATTRACTIVE FORCE | DISTANCE BETWEEN BOTH ENDS IN CASE OF CYCLIC STRUCTURE | SIDE CHAIN-RELATED TERM |

610

CALCULATION TARGET

MAIN CHAIN PARTICLE i+1    MAIN CHAIN PARTICLE i+1

$\theta$    $\theta'$

MAIN CHAIN PARTICLE i    MAIN CHAIN PARTICLE i

MAIN CHAIN PARTICLE i-1    MAIN CHAIN PARTICLE i-1

CALCULATE DIFFERENCE AT SITE CHANGED BY STATE TRANSITION

620

CALCULATION TARGET

MAIN CHAIN PARTICLE i    MAIN CHAIN PARTICLE i+1    MAIN CHAIN PARTICLE i+1

$\tau$    PLANE B    MAIN CHAIN PARTICLE i    $\tau'$    PLANE B'

PLANE A    PLANE A

MAIN CHAIN PARTICLE i-1    MAIN CHAIN PARTICLE i-1

MAIN CHAIN PARTICLE i-2    MAIN CHAIN PARTICLE i-2

CALCULATE DIFFERENCE AT SITE CHANGED BY STATE TRANSITION

# FIG. 7

```
                                                                                    510
┌─────────────────────────────────────────────────────────────────────────────┐
│                          ENERGY CALCULATION                                    │
```

$$E = \Sigma_{angles} K_\theta \cdot (\theta - \theta_0) + \Sigma_{dihedral} K_\tau \cdot \left[1 + \cos(N(\tau - \tau_0))\right] + \Sigma_{i<j-3}\left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4 + E_{res}$$

| ANGLE | DIHEDRAL ANGLE | REPULSIVE OR ATTRACTIVE FORCE | DISTANCE BETWEEN BOTH ENDS IN CASE OF CYCLIC STRUCTURE | SIDE CHAIN-RELATED TERM |

710

**CALCULATION TARGET**

MAIN CHAIN
PARTICLE i+1

GROUP A        GROUP B

MAIN CHAIN
PARTICLE i

MAIN CHAIN
PARTICLE i-1

⇒

MAIN CHAIN
PARTICLE i+1        GROUP B

GROUP A

MAIN CHAIN
PARTICLE i

MAIN CHAIN
PARTICLE i-1

CALCULATE FOR PAIR OF MAIN CHAIN PARTICLES BELONGING
TO DIFFERENT GROUPS

720

**CALCULATION TARGET**

IN CASE OF CYCLIC
STRUCTURE

## FIG. 8

510

**ENERGY CALCULATION**

$$E = \sum_{angles} K_\theta \cdot (\theta - \theta_0) + \sum_{dihedral} K_\tau \cdot \left[1 + \cos\left(N(\tau - \tau_0)\right)\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4 + E_{res}$$

ANGLE  DIHEDRAL ANGLE  REPULSIVE OR ATTRACTIVE FORCE  DISTANCE BETWEEN BOTH ENDS IN CASE OF CYCLIC STRUCTURE  SIDE CHAIN-RELATED TERM

810

**CALCULATION TARGET**

$$E_{res} = K_{dup} \cdot \sum_{i,j(i\neq j)} \{dup(p(i), q(j)) + dup(q(i), q(j))\}$$

$$+ K_{adj} \cdot \sum_{i,j(i\neq j)} adj(p(i), q(j))$$

$$+ \sum_{i,j\in\{SSB\}} K_{ss} \cdot \left(dist(q(i), q(j)) - D_{ss}\right)^4$$

$$+ K_{inter} \cdot \sum_{i,j(i\neq j)} Inter(A(i), A(j))$$

- $K_{dup}$, $K_{adj}$, $K_{SS}$, $K_{inter}$: WEIGHTS OF PENALTIES
- p(i): COORDINATES OF i-TH MAIN CHAIN PARTICLE, q(j): COORDINATES OF j-TH SIDE CHAIN PARTICLE
- A(i): TYPE OF AMINO ACID OF i-TH RESIDUE (SUCH AS GLY OR CYS), A(j): TYPE OF AMINO ACID OF j-TH RESIDUE (SUCH AS GLY OR CYS)
- dup(p(i), q(j)): FUNCTION THAT TAKES 1 IF COORDINATES OF i-TH MAIN CHAIN PARTICLE COINCIDE WITH COORDINATES OF j-TH SIDE CHAIN PARTICLE AND TAKES 0 OTHERWISE
- adj(p(i), q(j)): FUNCTION THAT TAKES 1 IF i-TH MAIN CHAIN PARTICLE IS ADJACENT TO j-TH SIDE CHAIN PARTICLE AND TAKES 0 OTHERWISE
- dist(p(i), q(j)): DISTANCE BETWEEN i-TH MAIN CHAIN PARTICLES AND j-TH SIDE CHAIN PARTICLES
- SSB: SET OF RESIDUE PAIRS FORMING SS BONDS
- $D_{SS}$: TARGET VALUE OF DISTANCE BETWEEN SIDE CHAIN PARTICLES FORMING SS BOND
- Inter(A(i), A(j)): INTERACTION BETWEEN i-TH AMINO ACID RESIDUE AND j-TH AMINO ACID RESIDUE

FIG. 9

AMINO ACID
RESIDUE TYPE

AMINO ACID RESIDUE TYPE

# FIG. 10

1000

**420**

RELATIVE COORDINATES
⟨0⟩:(-1,-1,0)、⟨1⟩:(-1,1,0)、⟨2⟩:(1,-1,0)、⟨3⟩:(1,1,0)
⟨4⟩:(-1,0,-1)、⟨5⟩:(-1,0,1)、⟨6⟩:(1,0,-1)、⟨7⟩:(1,0,1)
⟨8⟩:(0,-1,-1)、⟨9⟩:(0,-1,1)、⟨10⟩:(0,1,-1)、⟨11⟩:(0,1,1)

**1010**

TERMINAL APPARATUS

TWO BEAD MODEL

**1011**
STATE VARIABLE INPUT UNIT

INITIAL STATE, RELATIVE COORDINATES

**1014**
EXECUTION INSTRUCTION UNIT

**1012**
ENERGY MODEL INPUT UNIT

E

SEARCH ITERATION COUNTS OR SEARCH EXECUTION VARIABLES

**1013**
SEARCH INSTRUCTION UNIT

SEARCH INSTRUCTION

SEARCH RESULT (MINIMUM ENERGY VALUE AND FIRST AND SECOND STATE VARIABLES)

**1020**
ISING APPARATUS

**510**

$$E = \sum_{angles} K_\theta \cdot (\theta - \theta_0) + \sum_{dihedral} K_\tau \cdot \left[1 + \cos(N(\tau - \tau_0))\right] + \sum_{i<j-3} \left[5\left(\frac{\sigma_{ij}}{r_{ij}}\right)^{12} - 6\left(\frac{\sigma_{ij}}{r_{ij}}\right)^{10}\right] + K_{end} \cdot (R_{th} - r_{1N})^4 + E_{res}$$

# FIG. 11

FIG. 12

1000

1010

TERMINAL APPARATUS

SEARCH INSTRUCTION

SEARCH RESULT (MINIMUM ENERGY VALUE AND FIRST AND SECOND STATE VARIABLES)

ISING APPARATUS

1150

ENERGY VALUE STORAGE UNIT

1160

STATE VARIABLE STORAGE UNIT

1020

1110

SEARCH UNIT

ENERGY VALUE

FIRST AND SECOND STATE VARIABLES (AFTER STATE TRANSITION)

1120

OPTIMUM SOLUTION IDENTIFICATION UNIT

[0,1,4,6,3,2,7]
[11,8,3,4,9,5, 7,10]

1140

ENERGY CALCULATION UNIT

1170

RELATIVE COORDINATE STORAGE UNIT

1130

STATE CONVERSION UNIT

○MAIN CHAIN PARTICLE 0:                        = (0,0,0)
○MAIN CHAIN PARTICLE 1: (0,0,0)   +(-1,-1,0)    = (-1,-1,0)
○MAIN CHAIN PARTICLE 2: (-1,-1,0)  +(-1,1,0)     = (-2,0,0)
○MAIN CHAIN PARTICLE 3: (-2,0,0)   +(-1,0,-1)    = (-3,0,-1)
○MAIN CHAIN PARTICLE 4: (-3,0,-1)  +(1,0,-1)     = (-2,0,-2)
○MAIN CHAIN PARTICLE 5: (-2,0,-2)  +(1,1,0)      = (-1,1,-2)
○MAIN CHAIN PARTICLE 6: (-1,1,-2)  +(1,-1,0)     = (0,0,-2)
○MAIN CHAIN PARTICLE 6: (0,0,-2)   +(1,0,1)      = (1,0,1)

○SIDE CHAIN PARTICLE 0: (0,0,0)    +(0,1,1)      = (0,1,1)
○SIDE CHAIN PARTICLE 1: (-1,-1,0)  +(0,-1,-1)    = (-1,-2,-1)
○SIDE CHAIN PARTICLE 2: (-2,0,0)   +(1,1,0)      = (-1,1,0)
○SIDE CHAIN PARTICLE 3: (-3,0,-1)  +(-1,0,-1)    = (-4,0,-2)
○SIDE CHAIN PARTICLE 4: (-2,0,-2)  +(0,-1,1)     = (-2,-1,-1)
○SIDE CHAIN PARTICLE 5: (-1,1,-2)  +(-1,0,1)     = (-2,1,-1)
○SIDE CHAIN PARTICLE 6: (0,0,-2)   +(1,0,1)      = (1,0,-1)
○SIDE CHAIN PARTICLE 6: (1,0,1)    +(0,1,-1)     = (1,1,-2)

EP 4 521 410 A1

# FIG. 13

# FIG. 14

```
     ┌─────────────────────────┐
     │    START OF STABLE      │
     │ CONFORMATION SEARCH     │
     │     PROCESSING          │
     └────────────┬────────────┘
                  │               ⌇ S1401
     ┌────────────▼────────────┐
     │ SET NUMBER OF RESIDUES AND RESIDUE │
     │         SEQUENCE        │
     └────────────┬────────────┘
                  │               ⌇ S1402
     ┌────────────▼────────────┐
     │ RANDOMLY DETERMINE INITIAL STATE OF N MAIN │
     │ CHAIN PARTICLES AND SIDE CHAIN PARTICLES   │
     │ (RANDOMLY DETERMINE FIRST AND SECOND       │
     │         STATE VARIABLES)                   │
     └────────────┬────────────┘
                  │               ⌇ S1403
     ┌────────────▼────────────┐
     │ INPUT RELATIVE COORDINATES, ENERGY │
     │ CALCULATION FORMULA, AND SEARCH ITERATION │
     │         COUNTS          │
     └────────────┬────────────┘
                  │               ⌇ S1404
     ┌────────────▼────────────┐
     │      SEARCH PROCESSING  │
     └────────────┬────────────┘
                  │               ⌇ S1405
     ┌────────────▼────────────┐
     │ RECEIVE MINIMUM ENERGY VALUE AND FIRST │
     │ AND SECOND STATE VARIABLES │
     └────────────┬────────────┘
                  │
     ┌────────────▼────────────┐
     │     END OF STABLE       │
     │ CONFORMATION SEARCH     │
     │     PROCESSING          │
     └─────────────────────────┘
```

# FIG. 15

START OF SEARCH
PROCESSING

S1501

CALCULATE ENERGY VALUE E FOR INITIAL STATE
AND STORE minE = E

S1502

CHANGE ANY VALUE IN FIRST STATE VARIABLE AND
UPDATE COORDINATES OF MAIN CHAIN PARTICLE

S1503

UPDATE COORDINATES OF SIDE CHAIN PARTICLES WITH
UPDATE OF COORDINATES OF MAIN CHAIN PARTICLES

S1504

CALCULATE ENERGY VALUE E' OF TWO BEAD MODEL
BASED ON CURRENT COORDINATES OF MAIN CHAIN
PARTICLES AND SIDE CHAIN PARTICLES

S1505

DETERMINATION PROCESSING

S1506

DOES PROCESSING
REACH SEARCH ITERATION COUNT FOR SIDE
CHAIN PARTICLES?    YES

NO    S1507

CHANGE SECOND STATE VARIABLE AND UPDATE
COORDINATES OF SIDE CHAIN PARTICLES

S1508

DOES PROCESSING
REACH SEARCH ITERATION COUNT FOR MAIN
CHAIN PARTICLES?    NO

YES

RETURN

# FIG. 16

START OF DETERMINATION PROCESSING

S1601

(E'-E) < -Tlog(a)?

NO

YES

S1602

ADOPT FIRST AND SECOND STATE VARIABLES AFTER TRANSITION, AND UPDATE ENERGY VALUE

S1603

REJECT SECOND STATE VARIABLE AFTER TRANSITION

S1604

E < minE?

NO

YES

S1605

STORE minE = E AND STORE CORRESPONDING FIRST AND SECOND STATE VARIABLES AS OPTIMUM FIRST AND SECOND STATE VARIABLES

RETURN

# FIG. 17

1710

| ITEM | FACE-CENTERED CUBIC LATTICE | KNIGHT'S WALK |
|---|---|---|
| NUMBER OF BITS | 296kbit | 1365kbit |
| RAM CAPACITY | 325Gbyte | 6.8Tbyte |

⇩

1720

| ITEM | FACE-CENTERED CUBIC LATTICE | KNIGHT'S WALK |
|---|---|---|
| NUMBER OF BITS | 468bit | 936bit |
| RAM CAPACITY | 156byte | 156byte |

Europäisches
Patentamt

European
Patent Office

Office européen
des brevets

# EUROPEAN SEARCH REPORT

Application Number

EP 24 19 3053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 265 030 A (SKOLNICK JEFFREY [US] ET AL) 23 November 1993 (1993-11-23) * Abstract; Summary of the invention; Detailed description of the invention; figures 4,5, 15A-15F * | 1-10 | INV. G16B15/20 |
| X | US 2020/327955 A1 (SATO HIROYUKI [JP]) 15 October 2020 (2020-10-15) * paragraphs [0098] - [0101], [0112], [0187] - [0194] * | 1-10 | |
| X | US 2021/383897 A1 (TERASHIMA CHIEKO [JP] ET AL) 9 December 2021 (2021-12-09) * paragraphs [0172] - [0175], [0186], [0415] - [0422], [0473], [0474] * | 1-10 | |
| X | TOMAS BABEJ ET AL: "Coarse-grained lattice protein folding on a quantum annealer", ARXIV.ORG, CORNELL UNIVERSITY LIBRARY, 201 OLIN LIBRARY CORNELL UNIVERSITY ITHACA, NY 14853, 2 November 2018 (2018-11-02), XP080941413, | 1,9,10 | |
| A | * Abstract; Section IV. Conclusions and future work; figures 1, 6, 7 * | 2-8 | |

TECHNICAL FIELDS SEARCHED (IPC)

G16B

The present search report has been drawn up for all claims

| Place of search | Date of completion of the search | Examiner |
|---|---|---|
| Munich | 9 January 2025 | Hendrikse, Natalie |

EPO FORM 1503 03.82 (P04C01)

## ANNEX TO THE EUROPEAN SEARCH REPORT
## ON EUROPEAN PATENT APPLICATION NO.

EP 24 19 3053

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2025

| Patent document cited in search report | | Publication date | Patent family member(s) | | Publication date |
|---|---|---|---|---|---|
| US 5265030 | A | 23-11-1993 | NONE | | |
| US 2020327955 | A1 | 15-10-2020 | CN | 111816257 A | 23-10-2020 |
| | | | EP | 3723094 A1 | 14-10-2020 |
| | | | JP | 7251281 B2 | 04-04-2023 |
| | | | JP | 2020173643 A | 22-10-2020 |
| | | | US | 2020327955 A1 | 15-10-2020 |
| US 2021383897 | A1 | 09-12-2021 | CN | 113764053 A | 07-12-2021 |
| | | | EP | 3920187 A1 | 08-12-2021 |
| | | | JP | 7547799 B2 | 10-09-2024 |
| | | | JP | 2021192199 A | 16-12-2021 |
| | | | US | 2021383897 A1 | 09-12-2021 |

EPO FORM P0459

For more details about this annex : see Official Journal of the European Patent Office, No. 12/82

**EP 4 521 410 A1**

**Patent documents cited in the description**

- JP 2021082165 A